(19) Europäisches Patentamt
European Patent Office
Office européen des brevets

(11) Publication number: **0 116 860 B1**

# (12) EUROPEAN PATENT SPECIFICATION

(45) Date of publication of patent specification: **11.07.90**

(51) Int. Cl.[5]: **C 12 N 15/00,** C 12 N 9/10, C 12 P 13/04 // C12R1/19

(21) Application number: **84100521.8**

(22) Date of filing: **19.01.84**

(60) **Divisional application 87116645 filed on 11.11.87.**

## (54) The cloning and utilization of aminotransferase genes.

(30) Priority: **21.01.83 GB 8301700**

(43) Date of publication of application:
**29.08.84 Bulletin 84/35**

(45) Publication of the grant of the patent:
**11.07.90 Bulletin 90/28**

(84) Designated Contracting States:
**BE CH DE FR GB IT LI NL SE**

(56) References cited:
**ANNUAL REVIEW OF BIOCHEMISTRY, vol. 47, 1978, Palo Alto, California, USA; H.E. UMBARGER:"Amino Acid Biosynthesis and its Regulation", pp. 533-606**

**JOURNAL OF BACTERIOLOGY, vol. 130, no. 1, April 1977, Washington, D.C., USA; D.H. GELFAND et al.:"Escherichia coli Mutants Deficient in the Aspartate and Aromatic Amino Acid Aminotransferares", pp.429-440**

**The file contains technical information submitted after the application was filed and not included in this specification**

(73) Proprietor: **THE NUTRASWEET COMPANY**
**1751 Lake Cook Road**
**Deerfield Illinois 60015 (US)**

(72) Inventor: **Primrose, Sandy Blackadder**
**57 Friars Gardens Hughenden Valley**
**High Wycombe Buckinghamshire (GB)**
Inventor: **Edwards, Richard Mark**
**6 Gladstone Rise**
**High Wycome Buckinghamshire (GB)**

(74) Representative: **Werner, Hans-Karsten, Dr. et al**
**Deichmannhaus am Hauptbahnhof**
**D-5000 Köln 1 (DE)**

(56) References cited:
**MOLECULAR AND GENERAL GENETICS, vol. 181, no. 4, 1981, Springer-Verlag, Berlin-Heidelberg; L. CHRISTIANSEN et al.:"Cloning, Restriction Endonuclease Mapping and Post-Transcriptional Regulation of rpoA, the Structural Gene for Ribosomal Protein S1", pp. 548-551**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 116 860 B1

Courier Press, Leamington Spa, England.

**Description**

Background of the Invention

Field of the Invention

This invention involves the construction and use of genetically engineered plasmids containing the *tyr*B gene. These plasmids are constructed using restriction endonucleases to contain the genes coding for said aminotransferase enzyme used during the synthesis of amino acids. These plasmids promote the synthesis of aminotransferases in bacterial cells. This synthesis allows increased yields of aminotransferase enzymes and the elimination of the aminotransferase reaction as a rate-limiting step in amino acid biosynthesis.

Description of the Prior Art

The transmination of amino acid precursor molecules by aminotransferase enzymes is reviewed by Umbarger (Ann. Rev. Biochemistry, *47*: 533—606, 1978. Umbarger at p. 534 indicates he is reviewing principally the gram-negative bacteria *Escherichia coli* and *Salmonella typhimurium*, but that their pattern of amino acid synthesis and regulation is not always the universal pattern. Aminotransferase and transaminase are used as equivalent terms in this document. Present aminotransferase nomenclature defining the transaminases A, B and C and the evolution of this nomenclature is discussed by Umbarger at p. 550—552 and p. 581—582. To avoid the potential confusion of the transaminase A, B, C designation we will use the genetic terminology of *asp*C, *tyr*B and *ilv*E gene products as defined by Umbarger at p. 582. The *asp*C gene product will catalyze the transamination of amino acid precursors to produce aspartate, glutamate, phenylalanine and tyrosine. The *tyr*B gene product will catalyze the transamination of amino acid precursors to produce phenylalanine, tyrosine, glutamate, aspartate and leucine. The *ilv*E gene product will catalyze the transamination of the amino acid precursors to produce isoleucine, valine, leucine, phenylalanine, glutamate and alanine.

The location of the *asp*C, *tyr*B and *ilv*E genes on the genetic map of *E. coli* K12 strain is disclosed and discussed by Bachmann et al. (Microbiological Reviews, *44*: 1—56, March 1980). The *asp*C gene located at *E. coli* K12 map position 20 minutes codes for an enzyme here called aspartate aminotransferase and given the Enzyme Commission Number E.C. 2.6.1.1 (Bachmann et al, p. 4).

The *tyr*B gene located at *E. coli* K12 map position 91 minutes codes for an enzyme called tyrosine or aromatic aminotransferase and given the Enzyme Commission Number E.C. 2.6.1.5 (Bachmann, et al. p. 21). The *ilv*E gene located at *E. coli* K12 map position 84 minutes codes for an enzyme here called branched-chain-amino-acid aminotransferase and given Enzyme Commission Number E.C. 2.6.1.42 (Bachmann et al. p. 10).

These two publications by Umbarger and by Bachmann et al. are herein incorporated by reference.

Amino acid transamination by *asp*C and *tyr*B gene products are disclosed by Gelfand et al., (J. Bacteriology, *130*: 429—440, April 1977) to be responsible for essentially all the aminotransferase activity required for the *de novo* biosynthesis of tyrosine, phenylalanine, and aspartate in *E. coli* K12. However, the presence of the *ilv*E gene product alone can reverse a phenylalanine requirement indicating it has the ability to transaminate a precursor of phenylalanine.

Transducing phage lambda has been used to carry DNA fragments for *E. coli* in the *asp*C region (Christensen and Pedersen Mol. Gen Genet, *181*: 548—551, 1981). The *asp*C region in bacteriophage lambda was used as a source of the gene for the ribosomal protein S1. Their S1 gene was closed onto plasmids for research purposes. The *asp*C gene, however, was not cloned into a plasmid for the production of transaminases. The *asp*C region was used as a marker to facilitate isolation of a transducing phage in a tyrosine auxotroph lacking the *asp*C and *tyr*B genes. This transducing bacteriophage was not suitable for producing high levels of aminotransferases.

The problem to be solved is the cloning of the *tyr*B gene from *E. coli* and the use of the resulting plasmid in a process of increasing the rate of transamination of a substrate and thereby producing an amino acid.

Summary of the Invention

Applicant's invention describes the isolation of the gene coding for the *tyr*B aminotransferases use during the bacterial synthesis of amino acids and the construction of plasmids containing said aminotransferase gene.

Applicant's plasmids contain one or more copies of the *tyr*B gene. Applicant's invention describes a use for this aminotransferase gene in the synthesis of increased enzyme concentrations of the aminotransferase in bacterial cells. Applicant's invention describes a method for the production of increased amounts of aminotransferases. Applicant's invention describes a method of improved amino acid synthesis where the aminotransferase catalyzed reaction is rate limiting. Applicant's invention describes a method for the increased synthesis of L-phenylalanine wherein the aminotransferase catalyzed transamination of phenylpyruvic acid to phenylalanine is rate-limiting. Applicant's invention describes the nucleotide sequence of the *tyr*B gene encoding the enzyme aromatic aminotransferase. Applicant's invention describes the amino acid sequence of the aromatic aminotransferase encoded by the gene *tyr*B.

The aminotransferase gene *tyr*B codes for the synthesis of the aminotransferase which catalyze the transamination of the carbonyl precursors of amino acids.

The *tyr*B gene codes for tyrosine or aromatic aminotransferase (E.C. 2.6.1.5) and is catalytically active during the synthesis of phenylalanine, tyrosine, glutamate, aspartate and leucine.

When the transamination reaction in bacterial amino acid synthesis becomes rate-limiting, the presence of these plasmid-borne aminotransferase gene allows for the synthesis of additional aminotransferases. Aminotransferase synthesis sufficient to overcome the rate limitation results in an increased rate of amino acid biosynthesis.

These plasmids are used to increase the total quantity of aminotransferase present in a cell and it may be used as follows. The transamination reaction may be used within the cell, in a cell extract, or the extract can be utilized for further purification of the specific aminotransferase and then used in a purified state.

Objects of the Invention

It is an object of this invention to construct an extra-chromosomal element, that is a plasmid, characterized in that the plasmid contains a *tyr*B gene coding for the synthesis of an aminotransferase of the following amino acid sequence

N-MET-PHE-GLN-LYS-VAL-ASP-ALA-TYR-ALA-
GLY-ASP-PRO-ILE-LEU-THR-LEU-MET-GLU-ARG-PHE-LYS-GLU-ASP-PRO-ARG-SER-ASP-LYS-VAL-
ASN-LEU-SER-ILE-GLY-LEU-TYR-TYR-ASN-GLU-ASP-GLY-ILE-ILE-PRO-GLN-LEU-GLN-ALA-VAL-
ALA-GLU-ALA-GLU-ALA-ARG-LEU-ASN-ALA-GLN-PRO-HIS-GLY-ALA-SER-LEU-TYR-LEU-PRO-MET-
GLU-GLY-LEU-ASN-CYS-TYR-ARG-HIS-ALA-ILE-ALA-PRO-LEU-LEU-PHE-GLY-ALA-ASP-HIS-PRO-
VAL-LEU-LYS-GLN-GLN-ARG-VAL-ALA-THR-ILE-GLN-THR-LEU-GLY-GLY-SER-GLY-ALA-LEU-LYS-
VAL-GLY-ALA-ASP-PHE-LEU-LYS-ARG-TYR-PHE-PRO-GLU-SER-GLY-VAL-TRP-VAL-SER-ASP-PRO-
THR-TRP-GLU-ASN-HIS-VAL-ALA-ILE-PHE-ALA-GLY-ALA-GLY-PHE-GLU-VAL-SER-THR-TYR-PRO-
TRP-TYR-ASP-GLU-ALA-THR-ASN-GLY-VAL-ARG-PHE-ASN-ASP-LEU-LEU-ALA-THR-LEU-LYS-THP-
LEU-PRO-ALA-ARG-SER-ILE-VAL-LEU-LEU-HIS-PRO-CYS-CYS-HIS-ASN-PRO-THR-GLY-ALA-ASP-
LEU-THR-ASN-ASP-GLN-TRP-ASP-ALA-VAL-ILE-GLU-ILE-LEU-LYS-ALA-ARG-GLU-LEU-ILE-PRO-
PHE-LEU-ASP-ILE-ALA-TYR-GLN-GLY-PHE-GLY-ALA-GLY-MET-GLU-GLU-ASP-ALA-TYR-ALA-ILE-
ARG-ALA-ILE-ALA-SER-ALA-GLY-LEU-PRO-ALA-LEU-VAL-SER-ASN-SER-PHE-SER-LYS-ILE-PHE-
SER-LEU-TYR-GLY-GLU-ARG-VAL-GLY-GLU-LEU-SER-VAL-MET-CYS-GLU-ASP-ALA-GLU-ALA-ALA-
GLY-ARG-VAL-LEU-GLY-GLN-LEU-LYS-ALA-THR-VAL-ARG-ARG-ASN-TYR-SER-SER-PRO-PRO-ASN-
PHE-GLY-ALA-GLN-VAL-VAL-ALA-ALA-VAL-LEU-ASN-ASP-GLU-ALA-LEU-LYS-ALA-SER-TRP-LEU-
ALA-GLU-VAL-GLU-GLU-MET-ARG-THR-ARG-ILE-LEU-ALA-MET-ARG-GLN-GLU-LEU-VAL-LYS-VAL-
LEU-SER-THR-GLU-MET-PRO-GLU-ARG-ASN-PHE-ASP-TYR-LEU-LEU-ASN-GLN-ARG-GLY-MET-PHE-
SER-TYR-THR-GLY-LEU-SER-ALA-ALA-GLN-VAL-ASP-ARG-LEU-ARG-GLU-GLU-PHE-GLY-VAL-TYR-
LEU-ILE-ALA-SER-GLY-ARG-MET-CYS-VAL-ALA-GLY-LEU-ASN-THR-ALA-ASN-VAL-GLN-ARG-VAL-
ALA-LYS-ALA-PHE-ALA-ALA-VAL-MET-TER-C

and which upon transformation of a suitable host cell expresses said enzyme.

Yet another object of the invention is a process of increasing the rate of transamination of a susceptible substrate by the *tyr*B gene product set forth above comprising:

a) inserting a plasmid containing *tyr*B gene into a microorganism, b) expressing the *tyr*B gene in the microorganism resulting in the synthesis of an active aminotransferase, c) catalyzing the transamination of the susceptible substrate by the aminotransferase.

Another object of the invention is a method of increasing the rate of transamination of receptive amino acid precursor molecules accomplished by the insertion of a plasmid containing the *tyr*B gene into a microorganism, followed by the synthesis of a physiologically effective concentration of aminotransferase resulting in an increased rate of amino acid synthesis.

Still another object of the invention is a method of increasing the transamination of phenylpyruvic acid by the aminotransferases codes for by plasmids containing the *tyr*B genes and expressed in a microorganism.

Yet another object of the invention is a method of increasing the synthesis of an amino acid in a cell where the aminotransferase is a rate-limiting enzyme which is accomplished by the introduction and expression of a plasmid containing one or more *tyr*B aminotransferase genes.

Still another object of the invention is the increased synthesis of phenylalanine from phenylpyruvic acid by the introduction and expression of a plasmid containing a *tyr*B aminotransferase gene in a microorganism wherein the wild type transamination of phenylpyruvic acid is a rate-limiting step in the synthesis of phenylalanine.

Another object of the insertion is the increased synthesis of tyrosine from p-hydroxyphenylpyruvate by the introduction and expression of a plasmid containing a *tyr*B aminotransferase gene in a microorganism wherein the wild type transaminase of p-hydroxyphenylpyruvic acid is a rate-limiting step in the synthesis of tyrosine.

Still yet another object of the invention is the introduction and expression of a plasmid containing a *tyr*B aminotransferase gene in an enteric microorganism, one such microorganism being *Escherichia coli*.

Description of the Drawings

Figure 1 describes the biosynthetic pathway for the synthesis of the aromatic amino acids tyrosine, phenylalanine and tryptophan.

Figure 2 is a native protein gel electropherogram showing the presence or absence of the aminotransferases encoded by *tyr*B, *asp*C or *ilv*E genes in various bacterial strains.

Figure 3 describes the DNA nucleotide sequence of the *EcoRI-Bam*HI fragment carrying the *tyr*B gene.

Figure 4 describes the amino acid sequence of the aminotransferase encoded for by the *tyr*B gene.

Figure 5 illustrates an *E. coli* Flowchart showing intermediate strains and processes resulting in deposited strain HW159.

Figure 6 illustrates the modification of the *tyr*B+ plasmid.

Figure 7 illustrates the restriction map of the plasmid carrying the *asp*C gene.

Detailed Description of the Specific Embodiments

The aminotransferases or transaminases are a family of enzymes that covalently transfer an amino group from a donor molecule, such as aspartate or glutamate, to an acceptor α-keto-acid precursor of an amino acid, such as oxaloacetic acid. The reaction is freely reversible. Therefore, the aminotransferases can be utilized both in the synthesis and the degradation of amino acids.

Amino acid biosynthesis often requires transamination as the final step in biosynthesis. An example of such a process is the transamination of phenylpyruvic acid to produce phenylalanine with the simultaneous conversion of glutamate to α-ketoglutarate. This is shown as step 10 in Figure 1. The biosynthesis of the aromatic amino acids phenylalanine and tyrosine both require the action of an aminoatransferase as the final step. Phenylpyruvate is converted to phenylalanine by an aminotransferase that transfers an amino group from glutamate (figure 1, step 10). Similarly, an amino group from glutamate is transferred to 4-hydroxyphenylpyruvate to produce tyrosine (figure 1, step 12). Each aminotransferase has preferred substrates for the transamination reaction. However, residual activity is present on other substrates permitting each aminotransferase to participate in more than one amino acid's biosynthesis.

The *E. coli* transaminase encoded by the *tyr*B gene is active on phenylalanine, tyrosine, glutamate, aspartate and leucine.

It is recognized that other pathways exist for synthesizing tyrosine and phenylalanine in other microorganisms. One such pathway is found in cyanobacteria and *P. aeruginosa* and involves the conversion of prephenate directly to pretyrosine. Pretyrosine is then a substrate for conversion directly to tyrosine or to phenylalanine. It is recognized that analogous cloned transaminase genes for the appropriate transaminase are similarly useful in increasing the reaction synthesizing pretyrosine when there are excess levels of prephenate. This transaminase would result in increasing the rate of synthesis of tyrosine and phenylalanine.

The enzymatic steps in the synthesis of the aromatic amino acids are shown in figure 1, steps 1—20. Normally each enzymatic step is under regulation by controlling the synthesis of the enzyme and/or by allosterically regulating the rate of enzymatic catalysis. Wild type microorganisms normally do not over-produce any one amino acid due to these controls. Mutant strains in which the controls on the synthesis of a particular amino acid have been inactivated or by-passed may be isolated and in general these strains tend to over-produce that particular amino acid. In the case of L-phe, the regulation is of necessity complex, since part of its synthetic pathway is shared with the other aromatic amino acids L-tyrosine (L-tyr) and L-tryptophane (L-trp) (see Figure 1 of the accompanying drawings).

In *E. coli*, as in many microorganisms, the first step in the pathway is catalysed by the three DAHP synthetase isozymes each of which is sensitive to one of the three final products. In addition, the first steps in the pathway after the branch point at chorismate are also sensitive to feed-back inhibition. Anthranilate synthetase is inhibited by L-trp and the two chorismate mutase isozymes are inhibited by L-phe and L-tyr. The synthesis of a number of the enzymes concerned is also subject to regulation. The tyrosine repressor, the product of the *tyr*R gene, controls expression of DAHP synthetase (L-tyr) and chorismate mutase (L-tyr). The genes for these last two enzymes together constitute the tyrosine operon. The tryptophan repressor, the product of the *trp*R gene, controls expression of all the enzymes of the *trp* operon which convert chorismate to L-trp. This regulator molecule also controls the expression of DAHP synthetase (L-trp). In addition, expression of the *phe*A gene which codes for the chorismate mutase (L-phe) and the expression of the trp operon are rendered sensitive to the level of L-phe. The *trp* operon is rendered sensitive to the level of L-trp by an attenuator/leader peptide control system.

Mutant strains that over-produce amino acids are often isolated by selecting for resistance to appropriate amino acid analogues. In the case of L-phe production, one may isolate strains derepressed for DAHP (L-tyr) and DAHP (L-trp) expression by selecting 3-fluro-tyrosine and 5-methyl-tryptophan resistant mutants which generally have inactive *tyr*R and *trp*R repressors. Mutants in which the chorismate mutase (L-phe) is resistant to L-phe may be isolated by selecting for 2-thienyl-alanine resistance. A DAHP synthetase (L-phe) that is resistant to feed-back inhibition may be isolated by first constructing a mutant strain lacking the other two DAHP synthetase isozymes. In such a strain, the synthesis of L-tyr and L-trp is sensitive to the amount of L-phe in the medium. Mutants able to grow in medium containing L-phe, but lacking L-tyr or L-trp, usually contain feed-back resistant forms of DAHP synthetase (L-phe).

Now, a combination of these approaches will result in strains that over-produce L-phe. Since the synthetic pathway is largely shared with that of L-tyr and L-trp, attention must be given to the prevention of L-tyr and L-trp build-up if one is interested in the optimisation of L-phe synthesis. One simple expedient is to incorporate a *tyr*A mutation into an L-phe over-producing strain so as to prevent L-tyr accumulation and to avoid the unwanted diversion of precursors to L-phe. Similarly, a deletion of the entire trp operon would prevent accumulation of L-trp. Such mutants would, of course, have to be cultured in media that included these two amino acids so as to permit growth.

The combination of approaches outlined above results in a strain de-regulated for the synthesis of L-phe which accumulates that amino acid in significant amounts. Now, the rate limiting steps in a biosynthetic pathway are usually those at which control is exerted. In a de-regulated strain, it is likely that a new step will become rate-limiting. Thus, to increase the production of L-phe still further it is necessary to identify the new rate-limiting step and to increase the activity of the relevant enzyme. This may be done in one of three ways:

(a) by isolating mutant strains in which the relevant enzyme is present in greater amounts;

(b) by isolating mutant strains in which the relevant enzyme has a higher specific activity;

(c) achieving the same end as in (a), but by cloning the gene for the enzyme of interest onto a suitable multi-copy plasmid and introducing this into the L-phe over-producing strain.

If such an enzyme is not normally subject to regulation, isolating mutants of the first two classes may be difficult. The best way to achieve the increase in activity is to clone the gene for the relevant enzyme.

This is achieved by ligating DNA from a suitable donor strain carrying the gene for the enzyme one wishes to clone to a suitably prepared DNA from an appropriate vector. One can then transform a mutant strain lacking the activity of the newly cloned gene. The problem experimentally is to isolate the gene. If the lack of this activity bestows a recognizable phenotype on this recipient strain, such as auxotrophy for a particular amino acid, then one can simply select for clones carrying the gene of interest by virtue of the ability of such clones to complement the lesion in the recipient strain. As an alternative, the gene could be cloned first into a low copy number plasmid or into a suitable bacteriophage vector and subsequently sub-cloned into the desired multi-copy plasmid. Alternatively a strain carrying a suitable prophage near the gene of interest can be constructed and a specialized transducing phage carrying the desired gene isolated by inducing the lysogenic donor strain and using the resultant lysate to transduce the recipient to prototrophy. The gene of interest can then be sub-cloned from the DNA of this specialized transducing phage such as lambda.

Should the mutation of the gene of interest not confer any readily identifiable phenotype, such as auxotrophy, then the cloning of such a gene can be effected by cloning nearby genes for which a selection exists and then screening these clones for the presence of the gene of interest by assaying for the product of the desired gene after introducing the clones into a recipient deficient in that enzyme. Such an approach is facilitated by using methods that allow the cloning of large DNA fragments, such as those involving bacterio-phages or cosmids. If no readily selectable marker is known to be in the vicinity of the desired gene, then it may be possible to isolate a strain carrying the insertion of a readily selectable transposon, such as Tn 10, which encodes tetracycline resistance, near the gene of interest. Large DNA fragments containing this marker can then be cloned and the resultant phage or cosmids screened for the presence of the gene of interest. The gene could then be sub-cloned into a suitable high copy number plasmid.

The following are the strain of *E. coli* used in the examples. Beside each strain designation is a summary of the bacterial genotype and source. The relationship of intermediate strains of *E. coli* leading to the production of the auxotroph HW159 which is both *asp*C$^-$ and *tyr*B$^-$ is shown in Figure 5. The process which converted one strain to another is also summarized on the Flowchart in Figure 5.

Strain HW159, the *asp*C$^-$ and *tyr*B$^-$ mutant was deposited in The American Type Culture Collection, 12301 Parklawn Drive, Rockville, MD 20852, U.S.A. and has the number designation ATCC 39260.

TABLE 1

Strain List

| Strain | Genotype | Source |
|---|---|---|
| HW22 | metE | M. Edwards |
| BHB2688 | recA/λimm 434 CI857 b2 red 3 Eam15 Sam7 | J. Burke |
| BHB2690 | recA/λimm 434 CI857 b2 red 3 Dam15 Sam7 | J. Burke |
| E107 | thr1 leuB6 thyA6 thi1 dnaB107 deoC1 lacY1 tonA21 rpsL67 SupE44 | B. Bachmann |
| DG44 | hsdS thi1 lacY1 galK2 xyl5 mtl1 proA2 argE3 hisG4 hppT29 aspC13 rpsL31 tsx33 supE44 recB21 recC22 sbcB15 | B. Bachmann |
| MC1061 | araD139(ara-leu)del7697 (lacIPOZY)del74 galU galK hsdR rpsL | M. Casadaban |
| DG30 | thi1 proA2 argE3 hisG4 lacY1 galK2 ara14 xyl5 mtl1 rpsL31 tsx323 supE44 recB27 recC22 sbcB15 hsdS hppT29 ilvE12 tyrB509 aspC13 | B. Bachmann |
| ES430 | thi malB29 relA1 spoT1 | B. Bachmann |
| HW157 | araD139(ara-leu)del7697 (lacIPOZY)del74 galU galK hsdR rpsL aspC13 | This work |
| HW159 | araD139(ara-leu)del7697 (lacIPOZY)del74 galU galK hsdR rpsL aspC13 tyrB507 | This work (deposited) |
| HW225 | araD139(ara-leu)del7697 (lacIPOZY)del74 galU galK hsdR rpsL aspC13 tyrB507 recA srl: :Tn10 | This work |
| HW519 | Prototroph W3110 | Commercially Available |

EXAMPLES

Construction and use of Transaminase Plasmids

For the purpose of exemplification, a novel strain of *E. coli* that over-produces L-phe has been constructed. This was achieved by a multi-step process that involved systematically de-regulating the L-phe synthetic pathway. In such de-regulated strains, it has been found that the final step, namely the transamination of PPA to L-phe, becomes rate-limiting during fermentation and the PPA accumulates with detrimental effect. As a further example, therefore, the gene for the tyrosine (aromatic) amino-transferase from *E. coli* has been cloned onto a multi-copy plasmid. Introduction of this plasmid into the L-phe over-producing strain reduces this build-up of PPA and increases the efficiency of the fermentation. As an additional example, the gene for the aspartate aminotransferase of *E. coli* has been cloned onto a high copy number plasmid. This enzyme also has L-phe aminotransferase activity and incorporation of this plasmid in the L-phe over-producing strain also enhances the efficiency of the process.

Strains carrying plasmids with *tyr*B or *asp*C have added utility in that they over-produce the relevant enzyme to a considerable extent. They are therefore a novel and useful source of these enzymes.

The *tyr*B or *asp*C genes are also useful on a plasmid, in a suitable genetic background, by providing a selective pressure for maintenance of that plasmid. This selective pressure has advantages over conventional antibiotic selection methods.

Example 1

Preparation of Plasmid DNA

Plasmid DNA was prepared as follows (adapted from the method of J. Burke and D. Ish-Horowitz) (Nucleic Acids Research *9*: 2989—2998 (1981)). The desired bacterial strain was grown to saturation in 5 ml of L-Broth plus suitable antibiotics. The culture was transferred to a 15 ml corex tube and the cells deposited by centrifugation at 10,000 RPM for 1 minute. The supernatant was decanted and the pellet resuspended in 500 µl of fresh solution I, (50 mM D-glucose, 25 mM Tris pH 8.0, 10 mM EDTA and 2 mg Lysozyme ml-l) and then incubated at room temperature for 5 minutes. The cells were lysed by the addition of 1 ml fresh solution II (containing 0.2 M NaOH and 1% SDS). This was mixed in gently and then incubated on ice for 5 minutes. 750 µl of cold solution III (to 29 ml of glacial acetic acid add water to about 60 ml adjust the pH to 4.8 with 10 m KOH and make up to 100 ml) was then added and mixed in thoroughly. After a further 5 minutes on ice, the precipitated chromosonal DNA was pelleted by centrifugation at 10,000 rmp for 10 minutes. The supernatant was decanted to a fresh 15 ml corex tube and 3.75 ml of Ethanol was added. The tube was kept on ice for 15 minutes. The DNA was pelleted by centrifugation at 10,000 rpm for 10 minutes. The supernatant was then poured off and the pellet thoroughly resuspended in 10 mM Tris (ph 8.0), 1 mM EDTA, 20 µl of 3 M sodium acetate pH 7.0 was added and the DNA transferred to an Eppendorf micro-test tube. The DNA was extracted twice with 200 µl of ultra-pure phenol that contained 0.1% 8-hydroxy quinoline and had been pre-equilibrated twice against 1 mM Tris pH 8.0 and once against 100 mM Tris 10 mM EDTA pH 8.0. The residual phenol was removed by four extractions with 1 ml of diethyl ether. The DNA was then precipitated by the addition of 500 µl ethanol and incubation in a dry-ice/ethanol bath for 10 minutes. The DNA was pelleted by centrifugation at 10,000 rpm for 10 minutes. The supernatant was discarded and the pellet resuspended in 500 µl of 70% ethanol in water (v/v). The DNA was re-pelleted by centrifugation at 10,000 rpm for 10 minutes, the supernatant was discarded and the pellet containing the DNA was dried in a vacuum dessicator for 30 minutes. The DNA was redissolved finally in 50 µl of 10 mM Tris 1 mM EDTA and stored at −20°C.

This preparation generally gives about 5 µg of plasmid DNA when the strains used are derived from MC1061. The preparations also contain considerable amounts of RNA and so all the restriction digests described in this patent also include 100 µg ml-l RNASE A that has been pre-treated at 90° for 15 minutes to destroy DNAses. The preparation can also be scaled up to cope with plasmid isolation from 50 ml cultures.

Example 2

(a) Cloning of the *tyr*B gene

Isolation of cosmid clones carrying *tyr*B and DNA from the *E. coli* strain HW 22 (*met*E) was prepared according to the method of Marmur (PNAS 46:453, 1960). Aliquots of this DNA were partially digested with the restriction endonuclease *Sau*3A as follows: The DNA (80 µg) was made up to 400 µl in *Sau*3A buffer containing 50 mM NaCl, 6 mM Tris-HCl (tris (hydroxymethyl) aminomethane hydrochloride) pH 7.5, 5 mM $MgCl_2$, 100 µg/ml gelatin. The DNA solution was then split into 8 × 50 µl aliquots. *Sau*3A (New England Biolabs) was then serially diluted (2-fold steps) in *Sau*3A dilution buffer containing 50 mM KCl, 10 mM Tris-HCl pH 7.4, 0.1 mM EDTA (ethylene diamine tetra acetic acid, disodium salt), 1 mM dithiothreitol (DTT), 200 µg/ml bovine serum albumin (BSA) and 50% v/v glycerol. 5 µl of each of seven serial dilutions along with 5 µl of undiluted enzyme were then added to the DNA solution. The amount of enzyme added ranged from 2.5 to 0.02 u. The digestion was continued for 1 hour after which time the reactions were stopped by a five minute incubation at 65°C. The tubes were then cooled on ice.

Two µl aliquots of each of the digestions were taken and were subjected to electrophoresis on a 0.7% w/v agarose gel in TBE (50 mM tris-HCl pH 8.3, 50 mM boric acid and 1 mM EDTA) along with suitable markers. After staining with ethidium bromide (1 µg/ml) for 15 minutes, the gel was observed under 254 nm illumination to visualise the DNA. In this way, the sample giving the greatest amount of material in the 45 kb size range could be estimated.

The DNA from this sample was then ligated to DNA from the cosmid vector pHC79 which had been prepared as described below:

Two 25 µg aliquots of pHC 79 DNA were completely digested one with the restriction endonuclease *Hind III* (E.C. 3.2.12.21) and the other with the restriction endonuclease *Sal*I (E.C. 3.1.23.37). The *Hind III* digestion was carried out in 100 µl of *Hind III* digestion buffer containing 60 mM NaCl, 7 mM $MgCl_2$, 7 mM Tris-HCl pH 7.4, 100 µg/ml gelatin and 15 units of *Hind III* (New England Biolabs). Incubation was at 37°C for one hour. The enzyme was then inactivated by heating to 65°C for five minutes. The *Sal*I digestion was carried out in 100 µl of *Sal*I digestion buffer containing 150 mM NaCl, 6 mM Tris-HCl pH 7.9, 6 mM $MgCl_2$, 6 mM 2-mercaptoethanol, 100 µg/ml gelatin and 25 units of *Sal*I (New England Biolabs). Incubation was at 37°C for one hour. The enzyme was then inactivated by heating to 65°C for five minutes.

The two aliquots were pooled and digested with 10 units calf intestinal phosphatase (PL Labs) at 37°C for 2 hours to remove 5′ phosphates. The pooled pHC 79 was then adjusted to 0.3 M sodium acetate pH 7.0 and phenol extracted twice, followed by four ether extractions. The DNA was precipitated with 2.5 volumes of ethanol, washed with 70% v/v ethanol, dried and finally re-suspended in 10 mM Tris-HCl, 1 mM EDTA to a final concentration of 500 µg/ml. The ligation reactions contained 1 µg of prepared pHC 79 and 1 µg of *Sau*3A digested *E. coli* DNA in 8 µl of ligation buffer (10 mM Tris-HCl pH 7.9, 10 mM $MgCl_2$, 20 mM dithiothreitol, 25 µg/ml gelatin and 1 mM ATP). The reaction mixture was incubated at 37°C for 5 minutes, cooled and the ligation initiated by the addition of T4 DNA ligase (E.C. 6.5..1.1).

These reactions were continued for 4 hours at 15°C. Four µl aliquots of the ligation mixtures were then subjected to *in vitro* packaging into bacterio-phage λ particles. Strains BHB 2688 and BHB 2690 were inoculated from NZY agar plates into 50 ml of NZY broth (containing 10 g NZamine (from Humko Sheffield), 5 g yeast extract and 2.5 g NaCl/litre) and incubated with aeration at 30°C until the $E_{600}$ reached 0.3. The cultures were then raised to 45°C by immersion in a 60°C water bath and incubated at 45°C for 20 minutes without aeration. The two cultures were then vigorously aerated at 37°C for 3 hours. The two cultures were pooled, chilled to 4°C and harvested by centrifugation at 7000 rpm for 2 minutes. The pellet was washed once in 100 ml of cold M9 minimal medium (per liter: 6 g $Na_2HPO_4$, 3 g $KH_2PO_4$, 0.5 g NaCl, 1.0 $NH_4Cl$; adjusted to pH7 with 8 N NaOH, after autoclaving, sterile glucose added to 0.2% W/V, $CaCl_2$ added to 0.1 mM and $MgSO_4$ to 1 mM). The pellet was washed in 5 ml cold complementation buffer (40 mM Tris-HCl pH 8.0, 10 mM spermidine-HCl, 10 mM putrescine-HCl, 0.1% V/V 2-mercaptoethanol and 7% V/V dimethyl sulfoxide) and pelleted at 5000 rpm for 30 seconds. The cells were finally resuspended in 0.25 ml cold complementation buffer and dispensed into 20 µl aliquots in micro test tubes. These were frozen immediately in liquid nitrogen and stored at −80°C.

For the packaging reaction, one 20 µl packaging mix was removed from the liquid nitrogen and to it was immediately added 1 µl of 30 mM ATP. The mix was then placed on ice for 2 minutes. The 4 µl aliquots of ligated DNA were then added and mixed thoroughly. This was then incubated at 37°C for 30 minutes. After 30 minutes, 1 µl of 1 mg/ml DNAase (Worthington) was added and mixed in until the sample had lost its viscosity. To this packaged DNA preparation was then added 200 µl of the strain E 107 which had been grown to an $E_{600}$ of about 1.0 in tryptone maltose broth. $MgSO_4$ was also added to a final concentration of 10 mM. The packaged cosmids were absorbed for 30 minutes at 30°C after which time 500 µl of L-broth (Luria broth: 1% W/V bacto-tryptone, 0.5% W/V bacto-yeast extract, 0.5% W/V NaCl, 1.2% W/V glucose, pH 7) was added and the incubation continued for a further 30 minutes at 30°C. The cells were then plated on L-agar containing 100 µg/ml carbenicillin (α-carboxy benzyl penicillin) and incubated at 30°C for 24 hours.

Carbenicillin resistant colonies were then picked to L-agar carbenicillin plates and incubated overnight at 42°C. Strain E107 (3) carries the mutation *dna*B which renders it unable to grow at 42°C. Thus, only those colonies which had acquired a cosmid clone carrying *dna*B$^+$ from the wild-type donor should be able to grow at this temperature. In this way, 8 cosmid clones carrying *dna*B were isolated. Since *dna*B maps close to *tyr*B, the gene for the aromatic transaminase, and since cosmid cloning results in plasmids containing large inserts of donor DNA, it was reasonable to assume that some of the cosmid clones would also carry *tyr*B. That some of the clones did indeed carry *tyr*B was shown in the following way.

Cosmid DNA from each of the *dna*B clones was purified and packaged into bacteriophage λ particles as described above. The packaged cosmids were then introduced into the transaminase deficient strain DG 44 which carries the mutations *tyr*B and *asp*C. These two mutations together bestow a Tyr$^-$ Asp$^-$ phenotype on DG 44. Introduction of an intact *tyr*B gene would be expected to restore the ability of this strain to grow in the absence of tyrosine and aspartate. Direct screening of clones for *tyr*B is not easy in this strain, since it will not maintain plasmids derived from *col*E1, such as pHC 79. After introducing the cosmid clones into DG 44, therefore, 2 hours were allowed for recombination between the *tyr*B gene on the cosmid and the mutant allele on the bacterial chromosome. The cells were then washed and plated on minimal medium lacking tyrosine and aspartate to screen for the occurrence of *tyr*B$^+$ recombinants. Four of the original *dna*B clones gave *tyr*B$^+$ recombinants in this screen and must therefore carry at least some of the *tyr*B gene.

(b) Sub-cloning of restriction fragments from cosmid *dna*B clone

The *tyr*B cosmid clones were of limited utility owing to the large size thereof which resulted in a low copy number. Therefore, restriction fragments carrying the *tyr*B gene were sub-cloned into the multi-copy plasmid pAT153, (see for example, Twigg, A. J., and Shenatt, D., (1980), Nature, *283*, 216—218) as follows: Two µg aliquots of *dna*B cosmid number 5 which carries *tyr*B was digested in 20 µl reaction with the following restriction endonucleases *Bam*HI, *Hind*III, *Sal*I, *Sph*I, *Cla*I, *Eco*RI and *Bgl*II.

In each case, the incubation was at 37°C for one hour and the enzyme was inactivated by heating to 65°C for five minutes. For the *Bam*HI digestion, the reaction medium contained 150 mM NaCl, 6 mM Tris-HCl pH 7.9, 6 mM $MgCl_2$, 100 µg/ml gelatin and 4 units of *Bam*HI (New England Biolabs). Three (3) units of *Hind*III (New England Biolabs) were used in 60 mM NaCl, 7 mM $MgCl_2$, 7 mM Tris-HCl pH 7.4 and 100 µg/ml gelatin. In the case of *Sal*I, 5 units of enzyme obtained from New England Biolabs were used in 150 mM NaCl, 6 mM Tris-HCl pH 7.9, 6 mM $MgCl_2$, 7 mM 2-mercaptoethanol and 100 µg/ml gelatin. For *Sph*I, the reaction contained 50 mM NaCl, 6 mM Tris-HCl pH 7.4, 6 mM $MgCl_2$, 10 mM 2-mercaptoethanol, 100 µg/ml gelatin and 1 unit of the *Sph*I enzyme (New England Biolabs). The reaction mixture for *Cla*I digestion corresponded to that for *Hind*III, digestion, except that the enzyme used was 3 units of *Cla*I obtained from Boehringer Biochemicals. In the case of *Eco*RI 4.5 units of *Eco*RI (New England Biolabs) were used in 100 mM Tris-HCl pH 7.5, 50 mM NaCl, 5 mM $MgCl_2$ and 100 µg/ml gelatin. Lastly, the reaction medium for *Bgl*II

digestion contained 60 mM NaCl, 10 mM Tris-HCl pH 7.6, 10 mM $MgCl_2$, 10 mM 2-mercaptoethanol and 100 μg/ml gelatin.

These digested DNA preparations were then subjected to electrophoresis on a horizontal 1% w/v low gelling temperature (LGT) agarose gel in TBE for 6 hours at 5 V/cm. The DNA fragments were visualized by staining the gel for 15 minutes in a 1 μg/ml solution of ethidium bromide, followed by observation under a 366 nm UV light source. Individual bands were excised and stored at 4°C.

One (1) μg aliquots of pAT153 DNA were digested with the same set of restriction endonucleases in 20 μl reactions, except for *Bgl*II (which does not cut pAT153). Cosmid *Bgl*II fragments were sub-cloned into *Bam*HI-cut pAT153 (*Bam*HI and *Bgl*II give the same 5′ overhangs). After digestion, the linearized vector was digested with 1 unit of calf intestinal phosphatase (PL Labs) for 1 hour, to remove 5′ phosphates and prevent recircularization, incubated at 70°C for 10 minutes to inactivate the enzyme and then also run on a 1% w/v LGT agarose gel as described for the cosmid fragments. The band corresponding to plasmid linearized by each enzyme was then excised from the gel as described above.

The cosmid fragments were then ligated to the appropriately-cut vector as follows. The gel slices were melted by incubation at 65°C for 10 minutes and then cooled to 37°C. The melted gel slices were all about 100 μl. Two μl of vector fragment and 8 μl of a particular cosmid fragment were then added to 40 μl of pre-warmed 1.25 × ligation buffer (62.5 mM Tris-HCl pH 7.8, 12.5 mM $MgCl_2$, 25 mM dithiothreitol, 1.25 mM ATP and 62.5 mg/ml gelatin) and mixed thoroughly. Ligase was added and the reaction continued at 15°C overnight. The ligated samples were then re-melted at 65°C for 5 minutes, cooled to room temperature and added to 200 μl of competent cells of the *E. coli* strain HW 87 which had been prepared as follows. An overnight culture of HW 87 in L-broth was diluted 1:50 into 50 ml of fresh pre-warmed L-broth and incubated at 37°C with good aeration until the $E_{600}$ reached 0.6. The cells were then pelleted by centrifugation at 10,000 rpm for 5 seconds and resuspended in 25 ml of cold 50 mM $CaCl_2$. The cells were left on ice for 10 minutes after which they were re-pelleted as above and re-suspended in 2 ml of cold 50 mM $CaCl_2$. After a further 10 minute incubation at 0°C, the cells were competent for transformation.

After addition of the ligated DNA, the cells were incubated at 0°C for a further 10 minutes, heat-shocked at 37°C for 2 minutes and finally mixed with 750μl of pre-warmed L-broth. The cells were incubated for 30 minutes at 37°C to allow phenotypic expression of the plasmid encoded β-lactamase gene before plating suitable aliquots onto L-agar plates containing 200 μg/ml carbenicillin. The plates were incubated at 37°C overnight. Colonies containing recombinant plasmids were identified by the sensitivity thereof to tetracycline in the case of fragments cloned at the *Hind*III, *Sal*I, *Sph*I, *Bam*HI and *Cla*I sites of pAT 153.

Recombinants isolated using the restriction endonuclease *Eco*RI were identified by examining the size of the plasmids in individual colonies. This was performed as follows. Potential recombinant colonies were patched onto L-agar plates containing 200 μg/ml carbenicillin. These were incubated overnight at 37°C. The bacteria from about 1 $cm^2$ of each patch were re-suspended in lytic mix containing 10 mM Tris-HCl pH 8, 1 mM EDTA, 1% w/v sodium dodecyl sulfate (SDS), 2% w/v Ficoll and 0.5% W/V bromphenol blue (Sigma chemicals) 100 μg/ml RNAse. This was then incubated at 65°C for 30 minutes. Each sample was then vortex-mixed vigorously for 30 seconds. 50 μl aliquots of each preparation were then loaded onto a 1% w/v agarose gel and subjected to electrophoresis for 4 hours at 10 V/cm. The plasmid bands were stained with ethidium bromide as described above and visualized under 254 nm UV illumination. Recombinant plasmids were identified by the reduced mobility thereof compared to non-recombinant controls. Using these methods, a number of recombinant plasmids carrying various fragments from the original cosmid *dna*B clone were isolated. To facilitate the screening of these plasmids for those that carried the intact *tyr*B gene, a new strain carrying *tyr*B$^-$ and *asp*C$^-$ mutations was constructed.

## Example 3

Construction of transaminase deficient strain

It was decided to move the already characterised mutations in DG 44 into a background known to allow maintenance of the plasmids (that of strain MC 1061). Bacteriophage P1 was grown on a mixed culture of strain HW 22 carrying random insertion of the tetracycline resistant transposon Tn 10 prepared as follows: A saturated culture of HW 22 was grown overnight in lambda YTM broth (10 g bacto tryptone, 2.5 g NaCl and 0.2% w/v maltose per litre). This was diluted × 1/100 into 100 ml of fresh pre-warmed lambda YM broth and incubated at 37°C until the $OD_{600}$ reached 0.6. The cells were deposited by centrifugation at 10,000 rmp for five seconds and resuspended in 5 ml of lambda YM broth. NK 55, a bacteriophage lambda derivative carrying the tetracycline transposon Tn10, was then added to a multiplicity of infection of 0.2. The bacteriophage was absorbed for 45 minutes at 37°C. Then 200 ml aliquots were spread onto L-agar plates containing tetracycline (20 μg/ml) and sodium pyrophosphate (2.5 mM). The plates were incubated at 37°C for 24 hours. Approximately 5,000 $Tet^R$ colonies were pooled by washing the colonies off the plates using a total of 5 ml of L-broth. This was then diluted into 50 ml of L-broth containing 20 μg/ml tetracycline and grown overnight at 37°C. The Tn10 pool was stored at −20°C after adding sterile glycerol to bring the concentration to 20% w/v.

In order to grow bacteriophage P1 on the Tn10 pool, a 200 ml aliquot of this glycerol stock was diluted with 5 ml of L-broth including 10 μg/ml tetracycline and incubated overnight with shaking at 37°C. To 0.2 ml of this overnight culture was added 2 × $10^6$ plaque forming units (pfu) of phage P1 clear and 10 μl of 50 mM $CaCl_2$. The cells were incubated at 37°C for five minutes and then added to 5 ml of L-broth + 2.5 mM $CaCl_2$

pre-warmed to 37°C. The cells were then incubated at 37°C with vigorous aeration for four hours. The cell debris was removed by centrifugation at 10,000 rpm for five minutes and the phage-containing supernate stored over chloroform at 4°C.

This supernate was used to transduce DG 30 to permit growth on minimal medium lacking tyrosine and aspartate. (The minimal medium used was that of Vogel and Bonner; 0.2 g/l $MgSo_4.7H_2O$, 2 g/l citric acid, 10 g/l $K_2HPO_2$, 3.5 g/l $NaH_2PO_4.4H_2O$ and 10 mM $FeCl_2$. After autoclaving, glucose was added to a final concentration of 0.2% w/v along with necessary supplements of amino acids at 50 µg/ml. The $Tyr^+$ $Asp^+$ transductants were then replica-plated to the same medium including 10 µg/ml tetracycline to detect those transductants that had simultaneously become $Tet^R$. A number of these $Tet^R$ $Tyr^+$ $Asp^+$ recombinants were purified and used to prepare new P1 lysates. Each of these eight P1 preparations was then used to transduce DG 30 to $Tet^R$. From each experiment, 50 $Tet^R$ transductants were picked to minimal medium lacking tyrosine and aspartate to test for linkage between the site of the Tn 10 insertion involved and *asp*C or *tyr*B. From each of these experiments one $Tet^R$ colony which remained *tyr*B *asp*C was purified and again used to prepare a P1 lysate. These lysates were used to transduce MC 1061 to $Tet^R$. Cell extracts from these transductants were then run on native polyacrylamide gels and stained for L-phe transaminase activity as described by Gelfand.

The cell extracts were prepared by growing the desired transductants overnight in 10 ml of L-broth at 37°C. The cells were harvested by centrifugation at 10,000 rpm for fifteen seconds and re-suspended in 0.5—1.0 ml of sonication buffer depending upon the size of the pellet. The sonication buffer used was 25 mM Tris-HCl, 25 mM $KH_2PO_4$ pH 6.9, 0.2 mM pyridoxal phosphate, 0.5 mM dithiothreitol, 0.2 mM EDTA and 10% v/v glycerol. The cell suspensions were transferred to Eppendorf micro test tubes and kept on ice. Each suspension was sonicated with a Davies sonicator using four five-second bursts at setting 2. The sonicated suspensions were then centrifuged at 15,000 rpm for five minutes at 4°C to remove cell debris and the supernatants transferred to a fresh micro test tube. The cell extracts were then kept on ice until they could be loaded on a gel or stored at −20°C.

The native gel electrophoresis was performed as follows. The gel consisted of 8.5% w/v acrylamide, 0.227% w/v bis-acrylamide in 375 mM Tris-HCl pH 8.3. This acrylamide stock was de-gassed and polymerized by the addition of 0.05% w/v ammonium persulfate and 0.016% w/v TEMED. After polymerization, the gels were pre-run in 37.5 mM Tris-HCl pH 8.3 for at least one hour at 4°C. Prior to loading the samples, the buffer was changed to running buffer consisting of 76.7 mM glycine, 1 mM Tris-HCl pH 8.3. About 50 µg protein from each cell extract was then loaded and subjected to electrophoresis at 10 v/cm for from four to six hours at 4°C. The protein concentration in the cell extracts was determined using the Bio-Rad protein reagent method. The gels were stained for L-phe transaminase activity as follows. The gel was washed briefly in 100 mM phosphate buffer pH 7.5 and then immersed in 500 ml of fresh staining solution containing 12.5 mM α-ketoglutarate, 0.2 mM pyridoxal phosphate, 0.6 mM nitro blue tetrazolium, 0.2 mM phenazine methosulphate, 30 mM L-phe and 100 mM $K_2HPO_4$ pH 7.5 that had been pre-warmed to 37°C. The gel was shaken gently in the staining solution for one hour at 37°C in the dark. The gel was then washed in distilled water and left in distilled water until it could be photographed. (See figure 2.) This illustrates the presence or absence of detectable aminotransferases in various E coli strains.

MC 1061 extracts prepared in this way gave three staining bands characteristic of the *ilv*E, *asp*C and *tyr*B activities. By comparing the transaminase pattern of extracts prepared from the transductants with that of MC 1061 it was possible to detect MC 1061 recombinants lacking the characteristic *asp*C activity.

One mutant which was devoid of *asp*C activity was purified and named HW 109. This strain was then cured of the Tn10 transposon by the method of Bochner *et al* (J.Bact *143*:926). HW 109 was grown overnight in L-broth. Approximately $10^6$ cells per plate were spread onto Bochner selective medium which was prepared as follows: Solution A (Bacto-tryptone 10g, Bacto yeast extract 5 g, chlortetracycline HCl 50 mg, agar 15 g, water 500 ml) and solution B (NaCl 10 g, $NaH_2PO_4.H_2O$ 10 g, glucose 2 g, water 500 ml) were autoclaved separately for 20 min at 15 psi. The solutions were mixed and cooled to pouring temperature. 5 ml of $ZnCl_2$ (20 mM) and 6 ml of 2 mg $ml^{-1}$ fusaric acid were then added prior to pouring. The plates were incubated for 24 hr at 37° and the fusaric acid resistant colonies isolated and purified by re-streaking on the same medium. These isolates were then tested for sensitivity to tetracycline. One tetracycline sensitive derivative of HW 109 was chosen and named HW 157.

This approach did not, however, yield *tyr*B derivatives of HW 22. To isolate a strain with Tn 10 linked to *tyr*B, the procedure was as follows. Strain ES 430 carries a mutation in *mal*B that renders it unable to use maltose as a carbon source. The *mal*B is reasonably closely linked with *tyr*B. Therefore, ES 430 was transduced with P1 grown on the random Tn 10 pool in HW 22 described above, selecting for $Tet^R$ on maltose Maconkey agar plates supplemented with 10 µg/ml tetracycline.

P1 transductions were performed as follows. The recipient strain was grown in L-broth to an $^{OD}600$ of about 1.0. $CaCl_2$ was added to a final concentration of 2.5 mM. Phage P1 clear grown on the desired donor was then added at a multiplicity of insertion of between 0.2 and 1.0 (usually $10^8$ pfu P1 clear per ml of recipient). The cells were incubated at 37°C for fifteen minutes, centrifuged at 10,000 rpm for five seconds, washed in the original volume of 0.1 M citrate buffer pH 7.0 and finally re-suspended in citrate buffer. Suitable aliquots were then plated on the selective medium.

Transductants which had simultaneously become $Tet^R$ and $Mal^+$ were picked and purified. P1 lysates were then prepared from these strains and used to transduce DG 44 to $Tet^R$. The $Tet^R$ colonies derived from each of the eight P1 lysates were then patched onto minimal agar plates lacking aspartate and tyrosine. In one of these experiments, good linkage betweeen Tn 10 and *tyr*B was obtained. Therefore, one $Tet^R$ *tyr*$B^-$ recombinant from this experiment was isolated, a P1 lysate prepared from this recombinant and used to transduce HW 157 described above to $Tet^R$. Fifty of these transductants were then patched onto minimal agar supplemented with leucine, but lacking L-aspartate and L-phenylalanine. In this way, a *tyr*$B^-$ *asp*$C^-$ derivative of MC 1061 was detected. This strain was designated HW 158. Finally, HW 159, a tetracycline sensitive derivative of HW 158 was isolated as described above for the isolation of HW 157. HW 159 will not grow on minimal medium supplemented with leucine since it requires aspartate and tyrosine for growth unlike the parental strain MC 1061 which only requires leucine.

Example 4

Screening of sub-clones for ability to complement the *tyr*B lesion in HW 159

All the recombinant plasmids obtained by subcloning DNA fragments from the *dna*B cosmid clone (described above) were introduced into strain HW 159 by transformation selecting on L-agar supplemented with 200 µg/ml carbenicillin. These transformants were then streaked onto minimal medium supplemented only with leucine to test for the ability of the presence of genes carried by the plasmid to complement the *tyr*B lesion in HW 159. One sub-clone carrying a *Cla*I fragment from cosmid *dna*B clone number 5 was found to restore the ability of HW 159 to grow on minimal medium supplemented with leucine and thus to carry the *tyr*B gene.

Sequencing of the *tyr*B gene was performed by the method of Maxam and Gilbert. The sequence of the *tyr*B gene is shown in figure 3. Based upon this DNA sequence the amino acid sequence for the aminotransferase encoded for by *tyr*B could be determined using the genetic triplet codons. This amino acid sequence is shown in figure 4.

Example 5 (Reference)

Cloning of the *asp*C gene:

As a starting point for the cloning of the *asp*C gene, it was decided to use the specialized transducing phage lambda *asp*C2 obtained from M. Ono. The phage was prepared as follows. A culture of HW 76, which is a double lysogen carrying lambda *asp*C and lambda CI 857 Sam 7, was grown to an $OD_{600}$ of 0.6 at 30°C. The culture was then incubated at 45°C for fifteen minutes to induce the prophage and then shaken vigorously at 37°C for three hours. Cell lysis was completed by the addition of 0.5 ml of $CHCl_3$ and the cell debris removed by centrifugation at 10,000 rpm for ten minutes. The phage was precipitated by the addition of NaCl to 2.4% w/v and polyethylene glycol (average molecular weight 6,000) to 10% w/v. The phage was precipitated overnight at 4°C and then pelleted by centrifugation at 5,000 rpm for ten minutes. The phage pellet was gently re-suspended in 10 ml of phage buffer consisting of 10 mM Tris-HCl pH 7.5, 10 mM $MgSO_4$. The phage was pelleted by centrifugation for one hour at 40,000 rpm and finally re-suspended in 1 ml of phage buffer.

The phage was further purified by running on a CsCl block gradient as follows. The 1 ml of phage was applied to a two-step block gradient in cellulose nitrate tubes. The gradient contained 1 ml of 5M CsCl, 10 mM $MgSO_4$, 10 mM Tris-HCl pH 8.0 and 0.1 mM EDTA overlaid with 3 ml of 3M CsCl, 10 mM $MgSO_4$, 10 mM Tris-HCl pH 8.0 and 0.1 mM EDTA. The gradient was centrifuged in a Beckmann SW 65 rotor for one hour at 30,000 rpm at 20°C. The phage band was removed in 0.5 ml from the side of the tube using a 1 ml syringe with a 5/8 inch (1.6 cm) 25 gauge needle. The 0.5 ml of phage was then mixed with 0.5 ml of saturated CsCl solution (25°C) in 10 mM $MgSO_4$, 10 mM Tris-HCl pH 8.0 and 0.1 mM EDTA and mixed well in a cellulose nitrate centrifuge tube. This was then overlaid with 3 ml of 5M CsCl in 10 mM $MgSO_4$, 10 mM Tris-HCl pH 8.0 and 0.1 mM EDTA and then 1 ml of 3M CsCl in 10 mM $MGSO_4$, 10 mM Tris-HCl pH 8.0 and 0.1 mM EDTA. This was again centrifuged at 30,000 rpm for one hour at 20°C. The phage was again removed from the side of the tube in 0.5 ml as before.

The DNA was extracted from the phage particles as follows. To the 0.5 ml of phage in a 15 ml corex centrifuge tube was added 50µl of 2M Tris-HCl pH 8.5, 0.2 M EDTA and 0.5 ml of formamide was added. After 30 minutes, 0.5 ml of water was added and the DNA precipitated by the addition of 3 ml of ethanol. The DNA was pelleted by centrifugation for five minutes at 10,000 rpm. The supernate was discarded and the pellet rinsed with 70% v/v ethanol. The DNA was dried *in vacuo* and finally dissolved in TE (10 mM Tris-HCl and 1 mM EDTA pH 8.0) to give a DNA concentration of 500 µg/ml. The lambda *asp*C DNA was partially digested with the restriction endonuclease *Sau*3A as described above for *E. coli* DNA. The DNA from all of the digests were pooled and run on a 1% w/v low gelling temperature agarose gel in TBE. Also prepared and run on the same gel was 1 µg of pAT153 DNA completely digested with the restriction endonuclease *Bam*HI and calf intestinal phosphatase as described above. The DNA was visualized under 366 nm U.V. light as described above.

The bands corresponding to linearised pAT153 and the partially restricted lambda *asp*C DNA between 2.5 and 6kb were excised. The DNA was extracted from the gel as follows. The gel slices were melted at 65°C for five minutes in 15 ml convex tubes, cooled to 37°C and diluted with two volumes of water at 37°C. The agarose was then removed by the addition of an equal volume of phenol at 37°C and vortexing for three minutes. The phases were separated by centrifugation at 10,000 rpm for five minutes and the upper aqueous phases removed to clean tubes. Three phenol extractions were performed. The final supernate was then extracted four times by vortexing with an equal amount of diethyl ether, allowing the phases to separate and then removing the upper ether layer. The volume of the remaining aqueous phase was determined and 3M sodium acetate added to bring the salt concentration to 0.3 M. Yeast transfer RNA was added to a final concentration of 5 µg/ml as a carrier. The DNA was precipitated by the addition of 2.5 volumes of ethanol, overnight incubation at −20°C and centrifugation at 10,000 rpm for 30 minutes. The pellet was washed in 70% v/v ethanol, re-centrifuged at 10,000 rpm for 30 minutes and dried *in vacuo.* The DNA was then dissolved in TE to give a concentration of about 100 µg/ml. The partially restricted lambda *asp*C DNA was then ligated to *Bam*HI cut pAT153 by adding 2 µl of prepared lambda *asp*C DNA to 2 µl of prepared pAT153 DNA in 4µl of 2x ligation buffer (described above). This mixture was incubated at 37°C for five minutes, cooled to room temperature and the ligation started by the addition of 1 µl of T4 DNA ligase (New England Biolabs). The ligation was continued at 15°C for four hours. The entire ligation reaction mixture was then cooled on ice and added to 200 µl of competent cells prepared from strain HW 159 as described above. The transformation was performed as above and the cells plated on L-agar containing 200 µg/ml carbenicillin. The plates were incubated overnight at 37°C. About 5,000 colonies were obtained. These were then cooled, washed twice in 10 ml of 0.85% w/v NaCl and plated onto minimal agar containing leucine and carbenicillin, but lacking tyrosine and aspartate. Only HW 159 cells which carried a recombinant plasmid containing *asp*C could grow on this medium.

After incubation at 37°C for twenty-four hours, about 100 colonies were obtained. Some of these were purified by successive streakings on the same medium. That these contained a recombinant plasmid was confirmed by running single colony agarose gels as described above. That the recombinant plasmids in these strains indeed conferred the $Asp^+$ $Tyr^+$ phenotype was confirmed by isolating the plasmids, re-transforming into HW 159 and showing that these transformants had become $Asp^+$ $Tyr^+$. That the transformants had been altered by the presence of cloned *asp*C as opposed to *tyr*B was shown by running native acrylamide gels on cell-free extracts and staining for transaminase activity as described above. These experiments confirmed that the plasmids indeed carried an intact *asp*C gene.

The actual level of aminotransferase activity is measured in arbitrary units indicating relative activity. Relative aminotransferase activity on various strains containing the *tyr*B and *asp*C genes on plasmid pAT153 is shown in table 2. The assay was performed using a sigma R aspartate transaminase assay kit. (Sigma Technical Bulletin No. 56—UV, revised August 1980)

TABLE 2
Transaminase Levels Measured Using Aspartate As Substrate
(Sigma Aspartate Transaminase Assay Kit)

| | mg Protein Units/Min. | Relative Activity |
|---|---|---|
| MC 1061 | 40 | 1 |
| HW 157 | 25 | 0.6 |
| HW 158 | ND | — |
| HW 158 pHC79 | 15 | 0.37 |
| HW 158 ptyrB | 494 | 12.3 |
| HW 158 paspCl-1 | 1738 | 43.5 |

Example 6

Further Analysis of the *tyr*B Clone

The ClaI insert carrying *tyr*B is approximately 4.5 kb. A preliminary restriction map was constructed by the commonly used technique of double restriction endonuclease digestion followed by gel electrophoresis to analyse the restriction fragments obtained. In one orientation (see figure 6) the ClaI insert had EcoRI, BamHI and NruI sites in positions that facilitated the reduction in plasmid size by *in vitro* deletion. For example, the small EcoRI fragment was removed as follows. One µg of ptyrB DNA was digested to completion with EcoRI in a final volume of 20 µl. The enzyme was inactivated by incubation at 65° for 5 minutes. A 2 µl aliquot of this digest was then diluted into 50 µl of ligase buffer, one µl of T4 DNA ligase added and the DNA ligated for 16 hours at 15°. Ligation at this low DNA concentration leads predominantly to the re-circularisation of the plasmid without the small EcoRI fragement. The DNA was then transformed into HW 87 selecting for carbenicillin resistance as described above. Individual transformants were picked, purified and analysed on single colony lysate gels as described above. Most of the transformants contained plasmids that exhibited higher mobility than the parental plasmid ptyrB.

Plasmid DNA from several of these clones was isolated and the loss of the small EcoRI fragment confirmed by EcoRI digestion and analysis of these digests by electrophoresis on a 1% agarose gel. All the deleted plasmids gave just one band corresponding to the large EcoRI fragments of ptyrB. The parental plasmid gave the expected two bands.

The EcoRI deleted ptyrB was then subjected to a similar series of manipulations to remove the BamHI fragment and this EcoRI BamHI deleted plasmid was further subjected to deletion using the enzyme *NruI* in the following buffer. (100 mM NaCl 6 mM Tris pH 7.4 6 mM $MgCl_2$ 5 mM β-mercaptoethanol 100 μg/ml gelatin) All three plasmids were purified and used to transform HW 225 (*tyr*B *asp*C recA) to carbenicillin resistance. All the transformants simultaneously acquired the ability to grow on minimal medium in the absence of L-tyrosine and L-aspartate. This demonstrates that the *tyr*B gene is still intact on all three deleted plasmids.

The BamHI-EcoRI fragment from the doble deleted ptyrB was subjected to sequence analysis following the protocols described by Maxam and Gilbert (Maxam, A. M., and W. Gilbert, 1977, PNAS *51*: 382—389; Masam, A. M., and W. Gilbert, 1977, MIE *65*: CH 57 499—559, Part I). One substantial open reading frame was discovered that could code for a protein of 380 or 396 amino acids in length. The precise start point for translation is not known. There is a typical ribosome binding site about 10 nucleotides upstream from an in phase GTG (valine codon). Alternatively there is an in phase ATG (methionine) but this is not preceded by a typical ribosome binding site. The DNA sequence of the EcoRI-BamHI fragment and the amino-acid sequence deduced from it are presented in figures 3 and 4.

Map of paspC

The restriction map of the smallest *asp*C sub-clone (paspC 3—4) was elucidated by a combination of single and double restriction digests as described above see figure 7. The *asp*C sub-clones do not possess restriction sites that allow the insert to be excised cleanly since they were made by ligating Sau3A fragments into the BamHI site of pAT 153, a procedure which does not regenerate the BamHI site.

The paspC3—4 (pME98) was used to transform the *E. coli* prototroph HW 519 and during fermentation produced levels of transaminase equivalent to or better than that of paspCI-1. The *E. coli* HW 519 carrying pME98 has the deposit number ATCC 39501.

Example 6

Increased Yield of Amino Acids

In the reactions requiring aminotransferase enzymes in figure 1, reactions #10 and #13, presence of additional aminotransferase enzymes facilitates amino acid synthesis. This increased level of the aminotranferase enzymes synthesized by the *asp*C, *tyr*B or *ilv*E gene enables a cell to overcome a rate-limited reaction at the transamination step.

A plasmid of the present invention, carrying the *asp*C, *gyr*B or *ilv*E gene is inserted into a bacterial cell by techniques well known in biochemistry. This inserted gene produces a messenger RNA which catalyzes the synthesis of increased levels of aminotransferases when the bacterial cell is grown in a suitable media. The presence of these increased levels of aminotransferases catalyzes increased levels of amino acids. The amino acids are then harvested from the cells and media by purification procedures well known in fermentation science.

The addition of the *tyr*B containing plasmid into *E.Coli* 13281 (U.S. Patent 2,973,304) resulted in an 11% increase in L-phenylalanine production as shown in Table 3.

TABLE 3
Phenylalanine Production in *E. Coli*
(Strain 13281)

| | No Plasmid | Plus *tyr*B Plasmid |
|---|---|---|
| L-Phe Produced | 100% | 111% |

Culture grown at 32°C for 48 hours.

References Cited

*U.S. Patent Documents*
1. USP 2,973,304 Pfizer
2. USP 4,237,224 Cohen et al.

1. Umbarger, H. E. Annual Review of Biochemistry, *47*: 533—606, 1978.
2. Christiansen, L. and S. Pedersen, Mol. Gen. Genet (1981) *181*: 548—551.
3. Gelfand, D. H. and R. S. Steinberg, J. of Bacteriology, *130*: 429—440, April 1977.

4. Bachmann, B. J. and K. B. Low, Microbiological Reviews, *44*: 1—56, March, 1980.
5. Maxam, A. M. and W. Gilbert, P.N.A.S., *51*: 382—389, 1977.
6. Maxam, A. M. and W. Gilbert, MIE *65*: Chapter 57, 499—559 (Part I).
7. Sigma Technical Bulletin No. 56—UV, Revised August 1980 Sigma Chemical Co., St. Louis, MO 63178.

**Claims**

1. A plasmid comprising a replicating extra-chromosomal element characterized in that the plasmid contains a *tyr*B gene coding for the synthesis of an aminotransferase of the following amino acid sequence

N-MET-PHE-GLN-LYS-VAL-ASP-ALA-TYR-ALA-
GLY-ASP-PRO-ILE-LEU-THR-LEU-MET-GLU-ARG-PHE-LYS-GLU-ASP-PRO-ARG-SER-ASP-LYS-VAL-
ASN-LEU-SER-ILE-GLY-LEU-TYR-TYR-ASN-GLU-ASP-GLY-ILE-ILE-PRO-GLN-LEU-GLN-ALA-VAL-
ALA-GLU-ALA-GLU-ALA-ARG-LEU-ASN-ALA-GLN-PRO-HIS-GLY-ALA-SER-LEU-TYR-LEU-PRO-MET-
GLU-GLY-LEU-ASN-CYS-TYR-ARG-HIS-ALA-ILE-ALA-PRO-LEU-LEU-PHE-GLY-ALA-ASP-HIS-PRO-
VAL-LEU-LYS-GLN-GLN-ARG-VAL-ALA-THR-ILE-GLN-THR-LEU-GLY-GLY-SER-GLY-ALA-LEU-LYS-
VAL-GLY-ALA-ASP-PHE-LEU-LYS-ARG-TYR-PHE-PRO-GLU-SER-GLY-VAL-TRP-VAL-SER-ASP-PRO-
THR-TRP-GLU-ASN-HIS-VAL-ALA-ILE-PHE-ALA-GLY-ALA-GLY-PHE-GLU-VAL-SER-THR-TYR-PRO-
TRP-TYR-ASP-GLU-ALA-THR-ASN-GLY-VAL-ARG-PHE-ASN-ASP-LEU-LEU-ALA-THR-LEU-LYS-THP-
LEU-PRO-ALA-ARG-SER-ILE-VAL-LEU-LEU-HIS-PRO-CYS-CYS-HIS-ASN-PRO-THR-GLY-ALA-ASP-
LEU-THR-ASN-ASP-GLN-TRP-ASP-ALA-VAL-ILE-GLU-ILE-LEU-LYS-ALA-ARG-GLU-LEU-ILE-PRO-
PHE-LEU-ASP-ILE-ALA-TYR-GLN-GLY-PHE-GLY-ALA-GLY-MET-GLU-GLU-ASP-ALA-TYR-ALA-ILE-
ARG-ALA-ILE-ALA-SER-ALA-GLY-LEU-PRO-ALA-LEU-VAL-SER-ASN-SER-PHE-SER-LYS-ILE-PHE-
SER-LEU-TYR-GLY-GLU-ARG-VAL-GLY-GLU-LEU-SER-VAL-MET-CYS-GLU-ASP-ALA-GLU-ALA-ALA-
GLY-ARG-VAL-LEU-GLY-GLN-LEU-LYS-ALA-THR-VAL-ARG-ARG-ASN-TYR-SER-SER-PRO-PRO-ASN-
PHE-GLY-ALA-GLN-VAL-VAL-ALA-ALA-VAL-LEU-ASN-ASP-GLU-ALA-LEU-LYS-ALA-SER-TRP-LEU-
ALA-GLU-VAL-GLU-GLU-MET-ARG-THR-ARG-ILE-LEU-ALA-MET-ARG-GLN-GLU-LEU-VAL-LYS-VAL-
LEU-SER-THR-GLU-MET-PRO-GLU-ARG-ASN-PHE-ASP-TYR-LEU-LEU-ASN-GLN-ARG-GLY-MET-PHE-
SER-TYR-THR-GLY-LEU-SER-ALA-ALA-GLN-VAL-ASP-ARG-LEU-ARG-GLU-GLU-PHE-GLY-VAL-TYR-
LEU-ILE-ALA-SER-GLY-ARG-MET-CYS-VAL-ALA-GLY-LEU-ASN-THR-ALA-ASN-VAL-GLN-ARG-VAL-
ALA-LYS-ALA-PHE-ALA-ALA-VAL-MET-TER-C

and which upon transformation of a suitable host cell expresses said enzyme.

2. The plasmid of claim 1 characterized in that said *tyr*B gene comprises the following nucleotide sequence:

```
                                                                              ATT
                                                                              TAA
                                                                             STOP
ACATCACCGCAGCAAACGCCTTTGCCACACGTTGTACATTTGCCGTATTTAACCCGGCGACACACATGCGACCGCTGGCG
TGTAGTGGCGTCGTTTGCGGAAACGGTGTGCAACATGTAAACGGCATAAATTGGGCCGCTGTGTGTACGCTGGCGACCGC

ATGAGATAGACACCAAATTCTTCACGTAGTCGGTCAACCTGAGCGGCACTTAAACCGGTATAACTGAACATGCCGCGCTG
TACTCTATCTGTGGTTTAAGAAGTGCATCAGCCAGTTGGACTCGCCGTGAATTTGGCCATATTGACTTGTACGGCGCGAC

ATTAAGCAGATAATCGAAATTGCGTTCTGGCATCTCTGTGCTTAATACCTTCACCAATTCCTGACGCATTGCCAGAATGC
TAATTCGTCTATTAGCTTTAACGCAAGACCGTAGAGACACGAATTATGGAAGTGGTTAAGGACTGCGTAACGGTCTTACG

GAGTACGCATCTCTTCTACTTCCGCCAGCCAGCTGGCTTTCAATGCCTCGTCATTCAGCACTGCAGCCACCACCTGCGCA
CTCATGCGTAGAGAAGATGAAGGCGGTCGGTCGACCGAAAGTTACGGAGCAGTAAGTCGTGACGTCGGTGGTGGACGCGT

CCAAAATTCGGCGGGCTGGAGTAGTTGCGGCGAACTGTTGCTTTCAATTGCCCCAGTACCGGCCAGCGGCTTCGGCATC
GGTTTTAAGCCGCCCGACCTCATCAACGCCGCTTGACAACGAAAGTTAACGGGGTCATGCGCCGGTCGCCGAAGCCGTAG

TTCACACATAACAGAAAGTCCGCCGACGCGCTCGCCGTAAAGGGAGAAAATTTTCGAGAACGAATTGCTCACCAGAGCGG
AAGTGTGTATTGTCTTTCAGGCGGCTGCGCGAGCGGCATTTCCCTCTTTTAAAAGCTCTTGCTTAACGAGTGGTCTCGCC

GTAATCCAGCGCTGGCAATGGCGCGAATAGCGTAGGCATCCTCTTCCATACCGGCACCAAATCCTTGATAGGCAATATCG
CATTAGGTCGCGACCGTTACCGCGCTTATCGCATCCGTAGGAGAAGGTATGGCCGTGGTTTAGGAACTATCCGTTATAGC

AGGAATGGAATAAGCTCGCGGGCTTTGAGAATTTCAATCACCGCATCCCACTGATCATTAGTGAGATCGGCACCCGTTGG
TCCTTACCTTATTCGAGCGCCCGAAACTCTTAAAGTTAGTGGCGTAGGGTGACTAGTAATCACTCTAGCCGTGGGCAACC

GTTGTGGCAACATGGATGCAGCAACACAATACTGCGGGCAGGTAATGTTTTCAGCGTCGCCAACAGGTCATTAAAGCGCA
CAACACCGTTGTACCTACGTCGTTGTGTAATGACGCCCGTCCATTACAAAAGTCGCAGCGGTTGTCCAGTAATTTCGCGT

CGCCGTTAGTCGCTTCGTCATACCAGGGGTAAGTACTCACTTCGAATCCAGCCCCGGCGAATATTGCTACGTGGTTTTCC
GCGGCAATCAGCGAAGCAGTATGGTCCCCATTCATGAGTGAAGCTTAGGTCGGGGCCGCTTATAACGATGCACCAAAAGG

CAGGTAGGATCGCTGACCCAGACGCCTGATTCCGGGAAGTAGCGTTTCAGGAAATCCGCGCCCACTTTCAATGCCCCGGA
GTCCATCCTAGCGACTGGGTCTGCGGACTAAGGCCCTTCATCGCAAAGTCCTTTAGGCGCGGGTGAAAGTTACGGGGCCT

GCCGCCAAGGGTTTGAATGGTTGCTACGCGCTGTTGTTTCAGTACCGGATGGTCCGCACCAAACAGCAGCGGCGCAATGG
CGGCGGTTCCCAAACTTACCAACGATGCGCGACAACAAAGTCATGGCCTACCAGGCGTGGTTTGTCGTCGCCGCGTTACC

CATGGCGATAGCAGTTAAGCCCTTCCATCGGTAAATAAAGCGAAGCGCCATGAGGCTGCGCATTCAGGCGCGCTTCCGCC
GTACCGCTATCGTCAATTCGGGAAGGTAGCCATTTATTTCGCTTCGCGGTACTCCGACGCGTAAGTCCGCGCGAAGGCGG

TCCGCCACGGCTTGCAGTTGTGGAATAATTCCGTCTTCGTTGTAGTACAGACCGATACTTAAATTCACTTTGTCGCTGGG
AGGCGGTGCCGAACGTCAACACCTTATTAAGGCAGAAGCAACATCATGTCTGGCTATGAATTTAAGTGAAACAGCGACGC

AGGGTCTTCTTTAAAACGCTCCATAAGCGTAAGAATCGGGTCGCCAGCGTAGGCGTCAACTTTTTGAAACACGCGATGGT
TCCCAGAAGAAATTTTGCGAGGTATTCGCATTCTTAGCCCAGCGGTCGCATCCGCAGTTGAAAAACTTTGTGCGCTACCA
                                                                       START tyrB
TCTCC
AGAGG
```

3. A proces of increasing the rate of transamination of a susceptible substrate by the *tyr*B gene product set forth in claim 1 comprising:

a) inserting a plasmid containing *tyr*B gene into a microorganism, b) expressing the *tyr*B gene in the microorganism resulting in the synthesis of an active aminotransferase, c) catalyzing the transamination of the susceptible substrate by the aminotransferase.

4. The process of claim 3 wherein the said receptive substrate is phenylpyruvic acid.

5. A process using a microorganism for producing an amino acid wherein the amino acid transamination reaction becomes rate-limiting comprising the introduction into a microorganism of a plasmid according to claim 1 coding for an aminotransferase capable of transaminating the keto acid precursors of the amino acid.

6. Process of Claim 5 wherein the amino acid is L-phenylalanine and its keto-acid precursor is phenylpyruvic acid.

7. The process of claim 5 wherein the amino acid is tyrosine and its keto acid precursor is p-hydroxyphenylpyruvate.

8. Use of a plasmid of claim 1 in a process of increasing the rate of transamination of a susceptible substrate.

**Patentansprüche**

1. Ein sich replizierendes extra-chromosomales Element enthaltendes Plasmid, dadurch gekennzeichnet, daß das Plasmid ein für die Synthese einer Aminotransferase codierendes *try*B -Gen der folgenden Aminosäure-Sequenz

N-MET-PHE-GLN-LYS-VAL-ASP-ALA-TYR-ALA-

GLY-ASP-PRO-ILE-LEU-THR-LEU-MET-GLU-ARG-PHE-LYS-GLU-ASP-PRO-ARG-SER-ASP-LYS-VAL-

ASN-LEU-SER-ILE-GLY-LEU-TYR-TYR-ASN-GLU-ASP-GLY-ILE-ILE-PRO-GLN-LEU-GLN-ALA-VAL-

ALA-GLU-ALA-GLU-ALA-ARG-LEU-ASN-ALA-GLN-PRO-HIS-GLY-ALA-SER-LEU-TYR-LEU-PRO-MET-

GLU-GLY-LEU-ASN-CYS-TYR-ARG-HIS-ALA-ILE-ALA-PRO-LEU-LEU-PHE-GLY-ALA-ASP-HIS-PRO-

VAL-LEU-LYS-GLN-GLN-ARG-VAL-ALA-THR-ILE-GLN-THR-LEU-GLY-GLY-SER-GLY-ALA-LEU-LYS-

VAL-GLY-ALA-ASP-PHE-LEU-LYS-ARG-TYR-PHE-PRO-GLU-SER-GLY-VAL-TRP-VAL-SER-ASP-PRO-

THR-TRP-GLU-ASN-HIS-VAL-ALA-ILE-PHE-ALA-GLY-ALA-GLY-PHE-GLU-VAL-SER-THR-TYR-PRO-

TRP-TYR-ASP-GLU-ALA-THR-ASN-GLY-VAL-ARG-PHE-ASN-ASP-LEU-LEU-ALA-THR-LEU-LYS-THP-

LEU-PRO-ALA-ARG-SER-ILE-VAL-LEU-LEU-HIS-PRO-CYS-CYS-HIS-ASN-PRO-THR-GLY-ALA-ASP-

LEU-THR-ASN-ASP-GLN-TRP-ASP-ALA-VAL-ILE-GLU-ILE-LEU-LYS-ALA-ARG-GLU-LEU-ILE-PRO-

PHE-LEU-ASP-ILE-ALA-TYR-GLN-GLY-PHE-GLY-ALA-GLY-MET-GLU-GLU-ASP-ALA-TYR-ALA-ILE-

ARG-ALA-ILE-ALA-SER-ALA-GLY-LEU-PRO-ALA-LEU-VAL-SER-ASN-SER-PHE-SER-LYS-ILE-PHE-

SER-LEU-TYR-GLY-GLU-ARG-VAL-GLY-GLU-LEU-SER-VAL-MET-CYS-GLU-ASP-ALA-GLU-ALA-ALA-

GLY-ARG-VAL-LEU-GLY-GLN-LEU-LYS-ALA-THR-VAL-ARG-ARG-ASN-TYR-SER-SER-PRO-PRO-ASN-

PHE-GLY-ALA-GLN-VAL-VAL-ALA-ALA-VAL-LEU-ASN-ASP-GLU-ALA-LEU-LYS-ALA-SER-TRP-LEU-

ALA-GLU-VAL-GLU-GLU-MET-ARG-THR-ARG-ILE-LEU-ALA-MET-ARG-GLN-GLU-LEU-VAL-LYS-VAL-

LEU-SER-THR-GLU-MET-PRO-GLU-ARG-ASN-PHE-ASP-TYR-LEU-LEU-ASN-GLN-ARG-GLY-MET-PHE-

SER-TYR-THR-GLY-LEU-SER-ALA-ALA-GLN-VAL-ASP-ARG-LEU-ARG-GLU-GLU-PHE-GLY-VAL-TYR-

LEU-ILE-ALA-SER-GLY-ARG-MET-CYS-VAL-ALA-GLY-LEU-ASN-THR-ALA-ASN-VAL-GLN-ARG-VAL-

ALA-LYS-ALA-PHE-ALA-ALA-VAL-MET-TER-C

enthält und das bei Transformation einer geeigneten Wirtszelle das Enzym exprimiert.

2. Plasmid nach Anspruch 1, dadurch gekennzeichnet, daß das *tyr*B -Gen die folgende Nucleotid-Sequenz umfaßt:

```
                                                                         ATT
                                                                         TAA
                                                                         STOP
ACATCACCGCAGCAAACGCCTTTGCCACACGTTGTACATTTGCCGTATTTAACCCGGCGACACACATGCGACCGCTGGCG
TGTAGTGGCGTCGTTTGCGGAAACGGTGTGCAACATGTAAACGGCATAAATTGGGCCGCTGTGTGTACGCTGGCGACCGC

ATGAGATAGACACCAAATTCTTCACGTAGTCGGTCAACCTGAGCGGCACTTAAACCGGTATAACTGAACATGCCGCGCTG
TACTCTATCTGTGGTTTAAGAAGTGCATCAGCCAGTTGGACTCGCCGTGAATTTGGCCATATTGACTTGTACGGCGCGAC

ATTAAGCAGATAATCGAAATTGCGTTCTGGCATCTCTGTGCTTAATACCTTCACCAATTCCTGACGCATTGCCAGAATGC
TAATTCGTCTATTAGCTTTAACGCAAGACCGTAGAGACACGAATTATGGAAGTGGTTAAGGACTGCGTAACGGTCTTACG

GAGTACGCATCTCTTCTACTTCCGCCAGCCAGCTGGCTTTCAATGCCTCGTCATTCAGCACTGCAGCCACCACCTGCGCA
CTCATGCGTAGAGAAGATGAAGGCGGTCGGTCGACCGAAAGTTACGGAGCAGTAAGTCGTGACGTCGGTGGTGGACGCGT

CCAAAATTCGGCGGGCTGGAGTAGTTGCGGCGAACTGTTGCTTTCAATTGCCCCAGTACGCGGCCAGCGGCTTCGGCATC
GGTTTTAAGCCGCCCGACCTCATCAACGCCGCTTGACAACGAAAGTTAACGGGGTCATGCGCCGGTCGCCGAAGCCGTAG

TTCACACATAACAGAAAGTCCGCCGACGCGCTCGCCGTAAAGGGAGAAAATTTTCGAGAACGAATTGCTCACCAGAGCGG
AAGTGTGTATTGTCTTTCAGGCGGCTGCGCGAGCGGCATTTCCCTCTTTTAAAAGCTCTTGCTTAACGAGTGGTCTCGCC

GTAATCCAGCGCTGGCAATGGCGCGAATAGCGTAGGCATCCTCTTCCATACCGGCACCAAATCCTTGATAGGCAATATCG
CATTAGGTCGCGACCGTTACCGCGCTTATCGCATCCGTAGGAGAAGGTATGGCCGTGGTTTAGGAACTATCCGTTATAGC

AGGAATGGAATAAGCTCGCGGGCTTTGAGAATTTCAATCACCGCATCCCACTGATCATTAGTGAGATCGGCACCCGTTGG
TCCTTACCTTATTCGAGCGCCCGAAACTCTTAAAGTTAGTGGCGTAGGGTGACTAGTAATCACTCTAGCCGTGGCAACC

GTTGTGGCAACATGGATGCAGCAACACAATACTGCGGGCAGGTAATGTTTTCAGCGTCGCCAACAGGTCATTAAAGCGCA
CAACACCGTTGTACCTACGTCGTTGTGTAATGACGCCCGTCCATTACAAAAGTCGCAGCGGTTGTCCAGTAATTTCGCGT

CGCCGTTAGTCGCTTCGTCATACCAGGGGTAAGTACTCACTTCGAATCCAGCCCCGGCGAATATTGCTACGTGGTTTTCC
GCGGCAATCAGCGAAGCAGTATGGTCCCCATTCATGAGTGAAGCTTAGGTCGGGGCCGCTTATAACGATGCACCAAAAGG

CAGGTAGGATCGCTGACCCAGACGCCTGATTCCGGGAAGTAGCGTTTCAGGAAATCCGCGCCCACTTTCAATGCCCCGGA
GTCCATCCTAGCGACTGGGTCTGCGGACTAAGGCCCTTCATCGCAAAGTCCTTTAGGCGCGGGTGAAAGTTACGGGGCCT

GCCGCCAAGGGTTTGAATGGTTGCTACGCGCTGTTGTTTCAGTACCGGATGGTCCGCACCAAACAGCAGCGGCGCAATGG
CGGCGGTTCCCAAACTTACCAACGATGCGCGACAACAAAGTCATGGCCTACCAGGCGTGGTTTGTCGTCGCCGCGTTACC

CATGGCGATAGCAGTTAAGCCCTTCCATCGGTAAATAAAGCGAAGCGCCATGAGGCTGCGCATTCAGGCGCGCTTCCGCC
GTACCGCTATCGTCAATTCGGGAAGGTAGCCATTTATTTCGCTTCGCGGTACTCCGACGCGTAAGTCCGCGCGAAGGCGG

TCCGCCACCGCTTGCAGTTGTGGAATAATTCCGTCTTCGTTGTAGTACAGACCGATACTTAAATTCACTTTGTCGCTGGG
AGGCGGTGCCGAACGTCAACACCTTATTAAGGCAGAAGCAACATCATGTCTGGCTATGAATTTAAGTGAAACAGCGACGC

AGGGTCTTCTTTAAAACGCTCCATAAGCGTAAGAATCGGGTCGCCAGCGTAGGCGTCAACTTTTTGAAACACGCGATGGT
TCCCAGAAGAAATTTTGCGAGGTATTCGCATTCTTAGCCCAGCGGTCGCATCCGCAGTTGAAAAACTTTGTGCGCTACCA
                                                                      START tyrB
TCTCC
AGAGG
```

3. Verfahren zur Steigerung der Transaminierungs-Rate eines empfänglichen Substrats durch das *tyr*B -Gen-Produkt nach Anspruch 1, umfassend

a) das Einfügen eines *tyr*B -Gen enthaltenden Plasmids in einen Mikroorganismus;

b) das Exprimieren des *tyr*B -Gens in dem Mikroorganismus, was in der Synthese einer aktiven Aminotransferase resultiert;

c) das Katalysieren der Transaminierung des empfänglichen Substrats durch die Aminotransferase.

4. Verfahren nach Anspruch 3, worin das aufnehmende Substrat Phenylbrenztraubensäure ist.

5. Verfahren unter Verwendung eines Mikroorganismus zur Erzeugung einer Aminosäure, worin die Aminosäure-Transaminierungs-Reaktion geschwindigkeitsbegrenzend wird, umfassend die Einführung eines für eine Aminotransferase, die zur Transaminierung der Ketosäure-Vorstufen der Aminosäure befähigt ist, codierenden Plasmids in einen Mikroorganismus.

6. Verfahren nach Anspruch 5, worin die Aminosäure L-Phenylalanin ist und ihre Ketosäure-Vorstufe Phenylbrenztraubensäure ist.

7. Verfahren nach Anspruch 5, worin die Aminosäure Tyrosin ist und ihre Ketosäure-Vorstufe p-Hydroxyphenylpyruvat ist.

8. Verwendung eines Plasmids nach Anspruch 1 in einem Verfahren zur Steigerung der Transaminierungs-Rate eines empfänglichen Substrats.

## Revendications

1. Un plasmide comprenant un élément extrachromosomique de replication, caractérisé en ce que le plasmide contient un gène *tyr*B codant pour la synthèse d'une aminotransférase ayant la séquence d'aminoacides suivante:

N-MET-PHE-GLN-LYS-VAL-ASP-ALA-TYR-ALA-
GLY-ASP-PRO-ILE-LEU-THR-LEU-MET-GLU-ARG-PHE-LYS-GLU-ASP-PRO-ARG-SER-ASP-LYS-VAL-
ASN-LEU-SER-ILE-GLY-LEU-TYR-TYR-ASN-GLU-ASP-GLY-ILE-ILE-PRO-GLN-LEU-GLN-ALA-VAL-
ALA-GLU-ALA-GLU-ALA-ARG-LEU-ASN-ALA-GLN-PRO-HIS-GLY-ALA-SER-LEU-TYR-LEU-PRO-MET-
GLU-GLY-LEU-ASN-CYS-TYR-ARG-HIS-ALA-ILE-ALA-PRO-LEU-LEU-PHE-GLY-ALA-ASP-HIS-PRO-
VAL-LEU-LYS-GLN-GLN-ARG-VAL-ALA-THR-ILE-GLN-THR-LEU-GLY-GLY-SER-GLY-ALA-LEU-LYS-
VAL-GLY-ALA-ASP-PHE-LEU-LYS-ARG-TYR-PHE-PRO-GLU-SER-GLY-VAL-TRP-VAL-SER-ASP-PRO-
THR-TRP-GLU-ASN-HIS-VAL-ALA-ILE-PHE-ALA-GLY-ALA-GLY-PHE-GLU-VAL-SER-THR-TYR-PRO-
TRP-TYR-ASP-GLU-ALA-THR-ASN-GLY-VAL-ARG-PHE-ASN-ASP-LEU-LEU-ALA-THR-LEU-LYS-THP-
LEU-PRO-ALA-ARG-SER-ILE-VAL-LEU-LEU-HIS-PRO-CYS-CYS-HIS-ASN-PRO-THR-GLY-ALA-ASP-
LEU-THR-ASN-ASP-GLN-TRP-ASP-ALA-VAL-ILE-GLU-ILE-LEU-LYS-ALA-ARG-GLU-LEU-ILE-PRO-
PHE-LEU-ASP-ILE-ALA-TYR-GLN-GLY-PHE-GLY-ALA-GLY-MET-GLU-GLU-ASP-ALA-TYR-ALA-ILE-
ARG-ALA-ILE-ALA-SER-ALA-GLY-LEU-PRO-ALA-LEU-VAL-SER-ASN-SER-PHE-SER-LYS-ILE-PHE-
SER-LEU-TYR-GLY-GLU-ARG-VAL-GLY-GLU-LEU-SER-VAL-MET-CYS-GLU-ASP-ALA-GLU-ALA-ALA-
GLY-ARG-VAL-LEU-GLY-GLN-LEU-LYS-ALA-THR-VAL-ARG-ARG-ASN-TYR-SER-SER-PRO-PRO-ASN-
PHE-GLY-ALA-GLN-VAL-VAL-ALA-ALA-VAL-LEU-ASN-ASP-GLU-ALA-LEU-LYS-ALA-SER-TRP-LEU-
ALA-GLU-VAL-GLU-GLU-MET-ARG-THR-ARG-ILE-LEU-ALA-MET-ARG-GLN-GLU-LEU-VAL-LYS-VAL-
LEU-SER-THR-GLU-MET-PRO-GLU-ARG-ASN-PHE-ASP-TYR-LEU-LEU-ASN-GLN-ARG-GLY-MET-PHE-
SER-TYR-THR-GLY-LEU-SER-ALA-ALA-GLN-VAL-ASP-ARG-LEU-ARG-GLU-GLU-PHE-GLY-VAL-TYR-
LEU-ILE-ALA-SER-GLY-ARG-MET-CYS-VAL-ALA-GLY-LEU-ASN-THR-ALA-ASN-VAL-GLN-ARG-VAL-
ALA-LYS-ALA-PHE-ALA-ALA-VAL-MET-TER-C

et qui, par transformation dans une cellule hôte appropriée, exprime ladite enzyme.

2. Le plasmide selon la revendication 1, caractérisé en ce que ledit gène *tyr*B comprend la séquence de nucléotides suivante:

```
                                                                                ATT
                                                                                TAA
                                                                               STOP
ACATCACCGCAGCAAACGCCTTTGCCACACGTTGTACATTTGCCGTATTTAACCCGGCGACACACATGCGACCGCTGGCG
TGTAGTGGCGTCGTTTGCGGAAACGGTGTGCAACATGTAAACGGCATAAATTGGGCCGCTGTGTGTACGCTGGCGACCGC

ATGAGATAGACACCAAATTCTTCACGTAGTCGGTCAACCTGAGCGGCACTTAAACCGGTATAACTGAACATGCCGCGCTG
TACTCTATCTGTGGTTTAAGAAGTGCATCAGCCAGTTGGACTCGCCGTGAATTTGGCCATATTGACTTGTACGGCGCGAC

ATTAAGCAGATAATCGAAATTGCGTTCTGGCATCTCTGTGCTTAATACCTTCACCAATTCCTGACGCATTGCCAGAATGC
TAATTCGTCTATTAGCTTTAACGCAAGACCGTAGAGACACGAATTATGGAAGTGGTTAAGGACTGCGTAACGGTCTTACG

GAGTACGCATCTCTTCTACTTCCGCCAGCCAGCTGGCTTTCAATGCCTCGTCATTCAGCACTGCAGCCACCACCTGCGCA
CTCATGCGTAGAGAAGATGAAGGCGGTCGGTCGACCGAAAGTTACGGAGCAGTAAGTCGTGACGTCGGTGGTGGACGCGT

CCAAAATTCGGCGGGCTGGAGTAGTTGCGGCGAACTGTTGCTTTCAATTGCCCCAGTACGCGGCCAGCGGCTTCGGCATC
GGTTTTAAGCCGCCCGACCTCATCAACGCCGCTTGACAACGAAAGTTAACGGGGTCATGCGCCGGTCGCCGAAGCCGTAG

TTCACACATAACAGAAAGTCCGCCGACGCGCTCGCCGTAAAGGGAGAAAATTTTCGAGAACGAATTGCTCACCAGAGCGG
AAGTGTGTATTGTCTTTCAGGCGGCTGCGCGAGCGGCATTTCCCTCTTTTAAAAGCTCTTGCTTAACGAGTGGTCTCGCC

GTAATCCAGCGCTGGCAATGGCGCGAATAGCGTAGGCATCCTCTTCCATACCGGCACCAAATCCTTGATAGGCAATATCG
CATTAGGTCGCGACCGTTACCGCGCTTATCGCATCCGTAGGAGAAGGTATGGCCGTGGTTTAGGAACTATCCGTTATAGC

AGGAATGGAATAAGCTCGCGGGCTTTGAGAATTTCAATCACCGCATCCCACTGATCATTAGTGAGATCGGCACCCGTTGG
TCCTTACCTTATTCGAGCGCCCGAAACTCTTAAAGTTAGTGGCGTAGGGTGACTAGTAATCACTCTAGCCGTGGGCAACC

GTTGTGGCAACATGGATGCAGCAACACAATACTGCGGGCAGGTAATGTTTTCAGCGTCGCCAACAGGTCATTAAAGCGCA
CAACACCGTTGTACCTACGTCGTTGTGTTATGACGCCCGTCCATTACAAAAGTCGCAGCGGTTGTCCAGTAATTTCGCGT

CGCCGTTAGTCGCTTCGTCATACCAGGGGTAAGTACTCACTTCGAATCCAGCCCCGGCGAATATTGCTACGTGGTTTTCC
GCGGCAATCAGCGAAGCAGTATGGTCCCCATTCATGAGTGAAGCTTAGGTCGGGGCCGCTTATAACGATGCACCAAAAGG

CAGGTAGGATCGCTGACCCAGACGCCTGATTCCGGGAAGTAGCGTTTCAGGAAATCCGCGCCCACTTTCAATGCCCCGGA
GTCCATCCTAGCGACTGGGTCTGCGGACTAAGGCCCTTCATCGCAAAGTCCTTTAGGCGCGGGTGAAAGTTACGGGGCCT

GCCGCCAAGGGTTTGAATGGTTGCTACGCGCTGTTGTTTCAGTACCGGATGGTCCGCACCAAACAGCAGCGGCGCAATGG
CGGCGGTTCCCAAACTTACCAACGATGCGCGACAACAAAGTCATGGCCTACCAGGCGTGGTTTGTCGTCGCCGCGTTACC

CATGGCGATAGCAGTTAAGCCCTTCCATCGGTAAATAAAGCGAAGCGCCATGAGGCTGCGCATTCAGGCGCGCTTCCCCC
GTACCGCTATCGTCAATTCGGGAAGGTAGCCATTTATTTCGCTTCGCGGTACTCCGACGCGTAAGTCCGCGCGAAGGCGG

TCCGCCACGGCTTGCAGTTGTGGAATAATTCCGTCTTCGTTGTAGTACAGACCGATACTTAAATTCACTTTGTCGCTGGG
AGGCGGTGCCGAACGTCAACACCTTATTAAGGCAGAAGCAACATCATGTCTGGCTATGAATTTAAGTGAAACAGCGACGC

AGGGTCTTCTTTAAAACGCTCCATAAGCGTAAGAATCGGGTCGCCAGCGTAGGCGTCAACTTTTTGAAACACGCGATGGT
TCCCAGAAGAAATTTTGCGAGGTATTCGCATTCTTAGCCCAGCGGTCGCATCCGCAGTTGAAAAACTTTGTGCGCTACCA
                                                                         START tyrB
TCTCC
AGAGG
```

3. Un procédé pour accroître la vitesse de transamination d'un substrat sensible par le produit du gène *tyr*B mentionné dans la revendication 1, comprenant:

a) l'insertion d'un plasmide contenant le gène *tyr*B dans un microorganisme,

b) l'expression du gène *tyr*B dans le microorganisme résultant en la synthèse d'une aminotransférase active,

c) la catalyse de la transamination du substrat sensible par l'aminotransférase.

4. Le procédé selon la revendication 3, selon lequel ledit substrat récepteur est l'acide phénylpyruvique.

5. Un procédé utilisant un microorganisme pour la production d'un aminoacide selon lequel la réaction de transamination de l'aminoacide devient à vitesse limitée, comprenant l'introduction dans un microorganisme d'un plasmide selon la revendication 1 codant pour une aminotransférase capable de transaminer les précurseurs cétoacides de l'aminoacide.

6. Un procédé selon la revendication 5, selon lequel l'aminoacide est la L-phénylalanine et son précurseur cétoacide est l'acide phénylpyruvique.

7. Le procédé selon la revendication 5, selon lequel l'aminoacide est la tyrosine et son précurseur cétoacide est le p-hydroxyphénylpyruvate.

8. Utilisation d'un plasmide selon la revendication 1 dans un procédé pour accroître la vitesse de transamination d'un substrat sensible.

FIG. 1

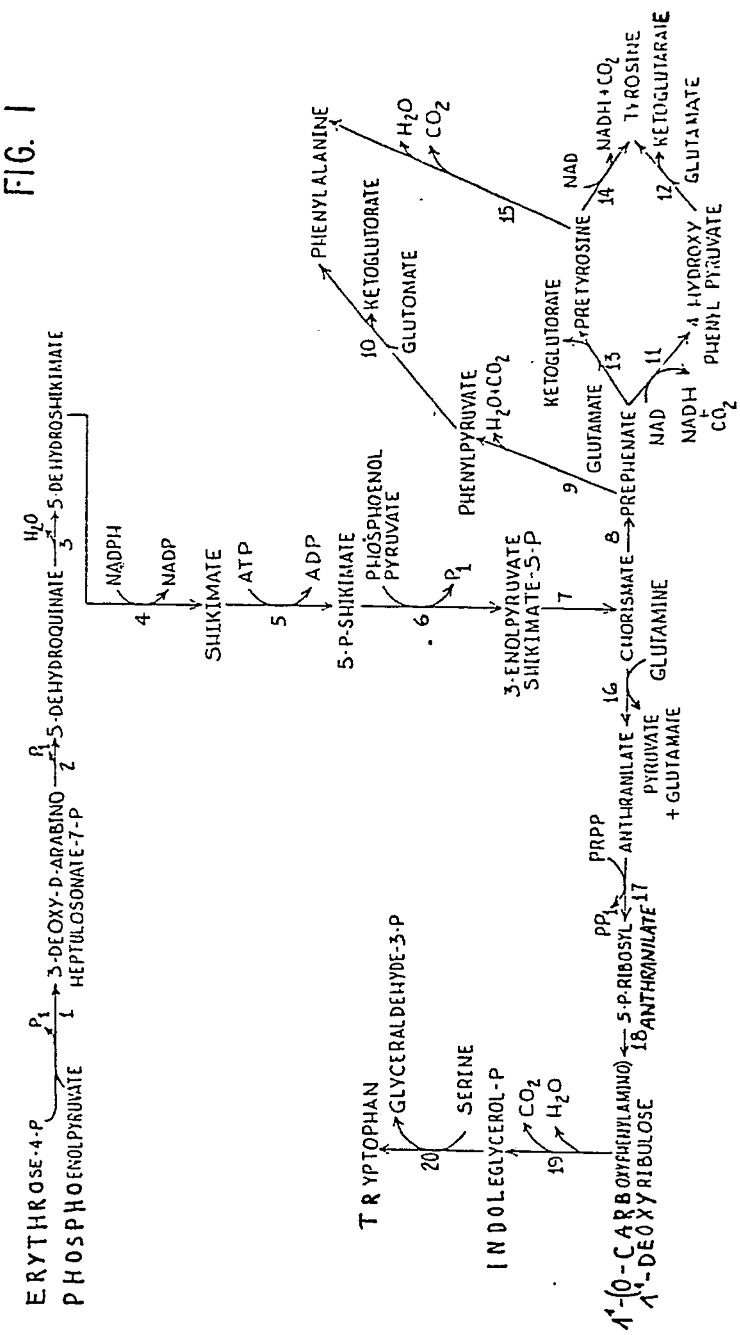


SYNTHESIS OF AROMATIC AMINO ACIDS

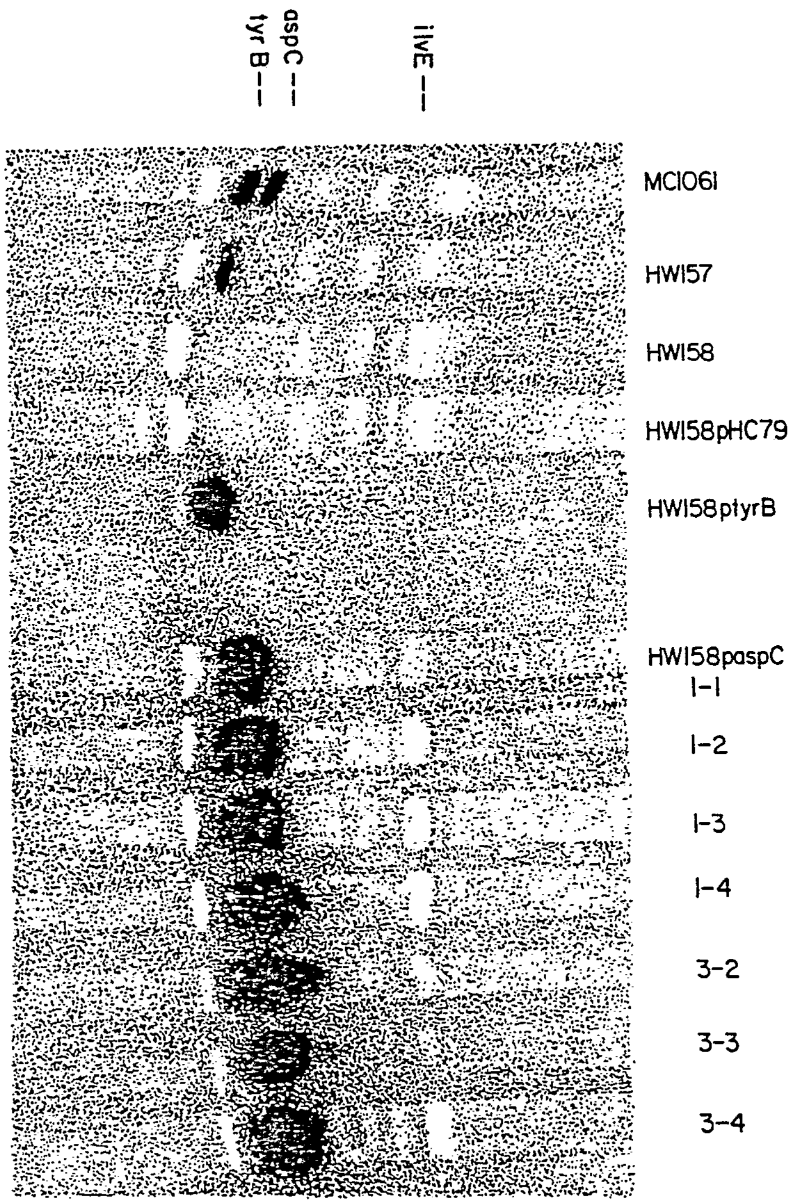


NATIVE PROTEIN GEL SHOWING AMINOTRANSFERASE SYNTHESIS

FIG. 2

```
GGATCCTGGTCATTTTATGGGGCGAAGGTTTGCCCGTAGAACGTATCGCTGAAATGACTA 60

AAGTAAGCGCTTACGAACTTATTACGCGCCTGACTTCAAGGTCGCGATGAAATACGTGG 120

ATTAATCGTTCTGTAATATTTGATTGTCTGTGCCGGATGCGGCGTGAATGCCTTATCCGG 180

CCAATAAAATCCTAAAAATTCAATAAGTTGATGTTCTTTCATGCTCTTATAAAGGTCGTG 240

CCTCTGGCGGATGTACGTTTGTCATGAGTCTCACTCTGTTGCTAATTGCCGTTCGCTCCT 300

GAACATCCACTCGATCTTCGCCTTCTTCCGGTTTATTGTGTTTTAACCACCTGCCCGTAA 360

                        V  F  Q  K  V  D  A  Y  A  G  D  P  I  L
ACCTGGAGAACCATCGCGTGTTTCAAAAAGTTGACGCCTACGCTGGCGACCCGATTCTTA 420

T  L  M  E  R  F  K  E  D  P  R  S  D  K  V  N  L  S  I  G
CGCTTATGGAGCGTTTTAAAGAAGACCCTCGCAGCGACAAAGTGAATTTAAGTATCGGTC 480

L  Y  Y  N  E  D  G  I  I  P  Q  L  Q  A  V  A  E  A  E  A
TGTACTACAACGAAGACGGAATTATTCCACAACTGCAAGCCGTGGCGGAGGCGGAAGCGC 540

R  L  N  A  Q  P  H  G  A  S  L  Y  L  P  M  E  G  L  N  C
GCCTGAATGCGCAGCCTCATGGCGCTTCGCTTTATTTACCGATGGAAGGGCTTAACTGCT 600

Y  R  H  A  I  A  P  L  L  F  G  A  D  H  P  V  L  K  Q  Q
ATCGCCATGCCATTGCGCCGCTGCTGTTTGGTGCGGACCATCCGGTACTGAAACAACAGC 660

R  V  A  T  I  Q  T  L  G  G  S  G  A  L  K  V  G  A  D  F
GCGTAGCAACCATTCAAACCCTTGGCGGCTCCGGGGCATTGAAAGTGGGCGCGGATTTCC 720

L  K  R  Y  F  P  E  S  G  V  W  V  S  D  P  T  W  E  N  H
TGAAACGCTACTTCCCGGAATCAGGCGTCTGGGTCAGCGATCCTACCTGGGAAAACCACG 780

V  A  I  F  A  G  A  G  F  E  V  S  T  Y  P  W  Y  D  E  A
TAGCAATATTCGCCGGGGCTGGATTCGAAGTGAGTACTTACCCCTGGTATGACGAAGCGA 840

T  N  G  V  R  F  N  D  L  L  A  T  L  K  T  L  P  A  R  S
CTAACGGCGTGCGCTTTAATGACCTGTTGGCGACGCTGAAAACATTACCTGCCCGCAGTA 900

I  V  L  L  H  P  C  C  H  N  P  T  G  A  D  L  T  N  D  Q
TTGTGTTGCTGCATCCATGTTGCCACAACCCAACGGGTGCCGATCTCACTAATGATCAGT 960

W  D  A  V  I  E  I  L  K  A  R  E  L  I  P  F  L  D  I  A
GGGATGCGGTGATTGAAATTCTCAAAGCCCGCGAGCTTATTCCATTCCTCGATATTGCCT 1020

Y  Q  G  F  G  A  G  M  E  E  D  A  Y  A  I  R  A  I  A  S
ATCAAGGATTTGGTGCCGGTATGGAAGAGGATGCCTACGCTATTCGCGCCATTGCCAGCG 1080

A  G  L  P  A  L  V  S  N  S  F  S  K  I  F  S  L  Y  G  E
CTGGATTACCCGCTCTGGTGAGCAATTCGTTCTCGAAAATTTTCTCCCTTTACGGCGAGC 1140

R  V  G  G  L  S  V  M  C  E  D  A  E  A  A  G  R  V  L  G
GCGTCGGCGGACTTTCTGTTATGTGTGAAGATGCCGAAGCCGCTGGCCGCGTACTGGGGC 1200

Q  L  K  A  T  V  R  R  N  Y  S  S  P  P  N  F  G  A  Q  V
AATTGAAAGCAACAGTTCGCCGCAACTACTCCAGCCCGCCGAATTTTGGTGCGCAGGTGG 1260

V  A  A  V  L  N  D  E  A  L  K  A  S  W  L  A  E  V  E  E
TGGCTGCAGTGCTGAATGACGAGGCATTGAAAGCCAGCTGGCTGGCGGAAGTAGAAGAGA 1320

M  R  T  R  I  L  A  M  R  Q  E  L  V  K  V  L  S  T  E  M
TGCGTACTCGCATTCTGGCAATGCGTCAGGAATTGGTGAAGGTATTAAGCACAGAGATGC 1380

P  E  R  N  F  D  Y  L  L  N  Q  R  G  M  F  S  Y  T  G  L
CAGAACGCAATTTCGATTATCTGCTTAATCAGCGCGGCATGTTCAGTTATACCGGTTTAA 1440

S  A  A  Q  V  D  R  L  R  E  E  F  G  V  Y  L  I  A  S  G
GTGCCGCTCAGGTTGACCGACTACGTGAAGAATTTGGTGTCTATCTCATCGCCAGCGGTC 1500

R  M  C  V  A  G  L  N  T  A  N  V  Q  R  V  A  K  A  F  A
GCATGTGTGTCGCCGGGTTAAATACGGCAAATGTACAACGTGTGGCAAAGGCGTTTGCTG 1560

A  V  H ***
CGGTGATGTAATGCAGGAAAGCAGGCTGGAGCTACCCAGCCTGCAGTGAAATTAAACTGT 1620

CGTCGCTTTCACTCTTTCTTTATAGATGATTTTTTTGATGCCATCGTTCTACGTGAGAGA 1680

TAATAAACGTTGTTAGTTCTTTTATTGTTAAGCTTATCCCAATTATCTG 1729

MOLECULAR WEIGHT OF PROTEIN= 43753.9
 398 AMINO ACIDS
```

FIG. 3

AROMATIC TRANSAMINASE AMINO ACID SEQUENCE

N-VAL-PHE-GLN-LYS-VAL-ASP-ALA-TYR-ALA-
GLY-ASP-PRO-ILE-LEU-THR-LEU-MET-GLU-ARG-PHE-LYS-GLU-ASP-PRO-ARG-SER-ASP-LYS-VAL-
ASN-LEU-SER-ILE-GLY-LEU-TYR-TYR-ASN-GLU-ASP-GLY-ILE-ILE-PRO-GLN-LEU-GLN-ALA-VAL-
ALA-GLU-ALA-GLU-ALA-ARG-LEU-ASN-ALA-GLN-PRO-HIS-GLY-ALA-SER-LEU-TYR-LEU-PRO-MET-
GLU-GLY-LEU-ASN-CYS-TYR-ARG-HIS-ALA-ILE-ALA-PRO-LEU-LEU-PHE-GLY-ALA-ASP-HIS-PRO-
VAL-LEU-LYS-GLN-GLN-ARG-VAL-ALA-THR-ILE-GLN-THR-LEU-GLY-GLY-SER-GLY-ALA-LEU-LYS-
VAL-GLY-ALA-ASP-PHE-LEU-LYS-ARG-TYR-PHE-PRO-GLU-SER-GLY-VAL-TRP-VAL-SER-ASP-PRO-
THR-TRP-GLU-ASN-HIS-VAL-ALA-ILE-PHE-ALA-GLY-ALA-GLY-PHE-GLU-VAL-SER-THR-TYR-PRO-
TRP-TYR-ASP-GLU-ALA-THR-ASN-GLY-VAL-ARG-PHE-ASN-ASP-LEU-LEU-ALA-THR-LEU-LYS-THP-
LEU-PRO-ALA-ARG-SER-ILE-VAL-LEU-LEU-HIS-PRO-CYS-CYS-HIS-ASN-PRO-THR-GLY-ALA-ASP-
LEU-THR-ASN-ASP-GLN-TRP-ASP-ALA-VAL-ILE-GLU-ILE-LEU-LYS-ALA-ARG-GLU-LEU-ILE-PRO-
PHE-LEU-ASP-ILE-ALA-TYR-GLN-GLY-PHE-GLY-ALA-GLY-MET-GLU-GLU-ASP-ALA-TYR-ALA-ILE-
ARG-ALA-ILE-ALA-SER-ALA-GLY-LEU-PRO-ALA-LEU-VAL-SER-ASN-SER-PHE-SER-LYS-ILE-PHE-
SER-LEU-TYR-GLY-GLU-ARG-VAL-GLY-GLU-LEU-SER-VAL-MET-CYS-GLU-ASP-ALA-GLU-ALA-ALA-
GLY-ARG-VAL-LEU-GLY-GLN-LEU-LYS-ALA-THR-VAL-ARG-ARG-ASN-TYR-SER-SER-PRO-PRO-ASN-
PHE-GLY-ALA-GLN-VAL-VAL-ALA-ALA-VAL-LEU-ASN-ASP-GLU-ALA-LEU-LYS-ALA-SER-TRP-LEU-
ALA-GLU-VAL-GLU-GLU-MET-ARG-THR-ARG-ILE-LEU-ALA-MET-ARG-GLN-GLU-LEU-VAL-LYS-VAL-
LEU-SER-THR-GLU-MET-PRO-GLU-ARG-ASN-PHE-ASP-TYR-LEU-LEU-ASN-GLN-ARG-GLY-MET-PHE-
SER-TYR-THR-GLY-LEU-SER-ALA-ALA-GLN-VAL-ASP-ARG-LEU-ARG-GLU-GLU-PHE-GLY-VAL-TYR-
LEU-ILE-ALA-SER-GLY-ARG-MET-CYS-VAL-ALA-GLY-LEU-ASN-THR-ALA-ASN-VAL-GLN-ARG-VAL-
ALA-LYS-ALA-PHE+ALA-ALA-VAL-MET-TER-C

FIGURE 4

# STRAIN FLOWCHART

MC1061

↓ P1 TRANSDUCTION

HW109 (aspC Tn10 MC1061)

↓ CURE Tn10

HW157 (aspC MC1061)

↓ P1 TRANSDUCTION

HW158 (aspC tyrB Tn10 MC1061)

↓ CURE Tn10

HW159 (aspC tyrB MC1061)

↓ P1 TRANSDUCTION

HW225 (aspC tyrB recA srl Tn10)

FIGURE 5

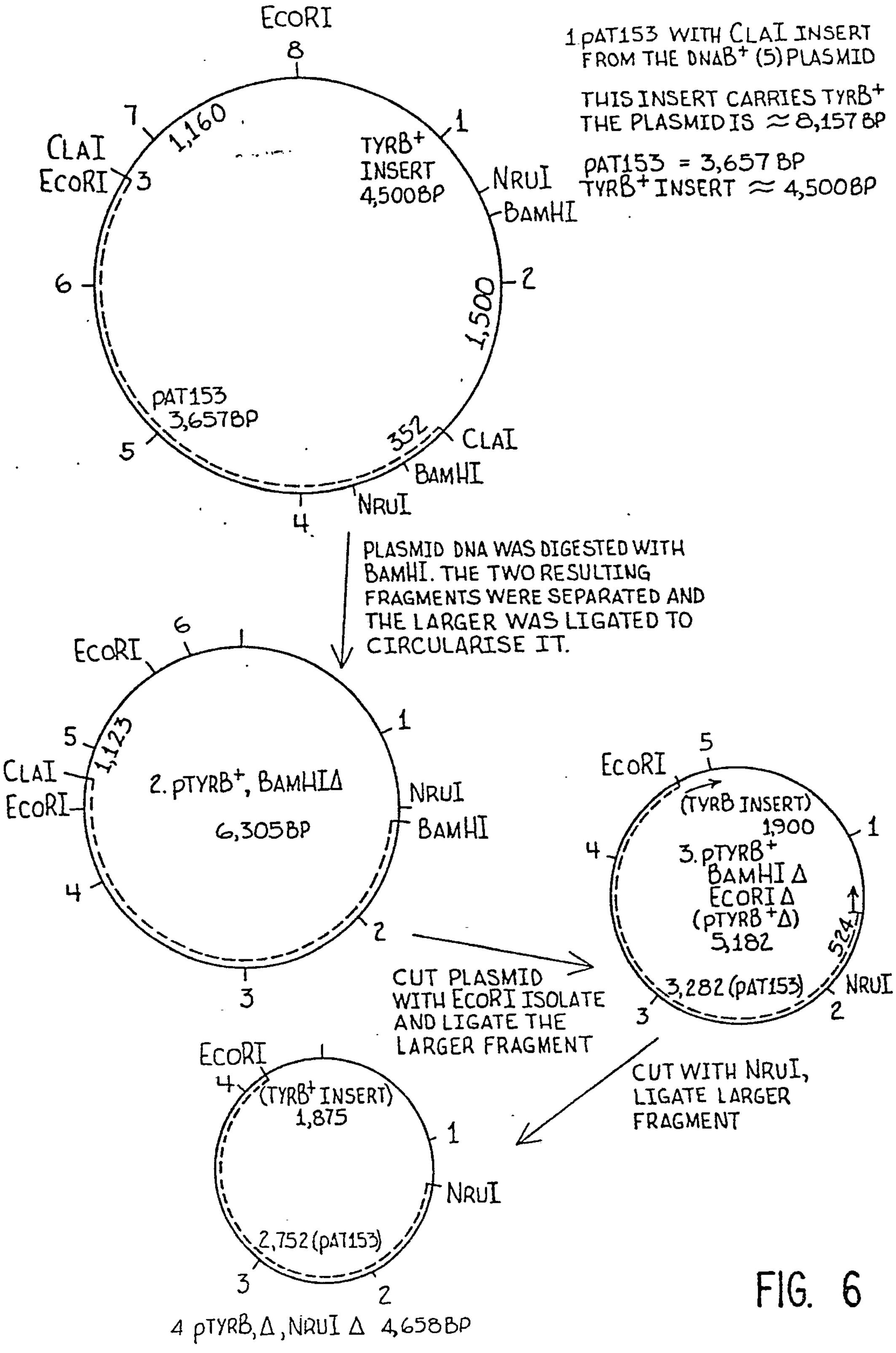


FIG. 6

MODIFICATION OF THE TYR B PLASMID

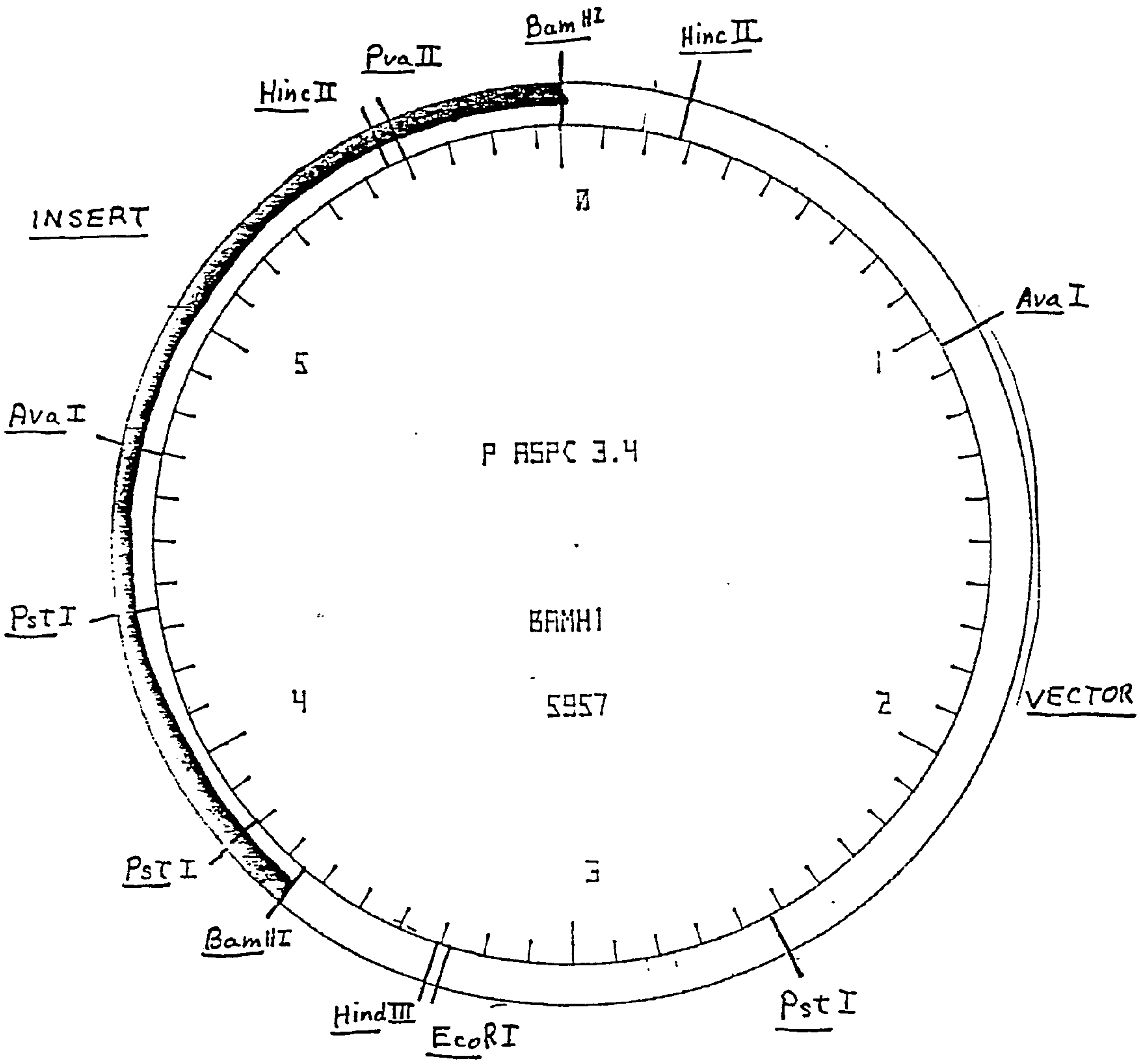
Bam HI
Hinc II
Pva II
Hinc II
INSERT
Ava I
Ava I
Pst I
Pst I
Bam HI
Hind III
EcoRI
Pst I
VECTOR
0
1
2
3
4
5
P ASPC 3.4
BAMH1
5957

FIGURE 7